# EUROPEAN PATENT SPECIFICATION

(11) **EP 3 006 450 B1**
(45) Date of publication and mention of the grant of the patent: **29.07.2020**
(21) Application number: 13886231.3
(22) Date of filing: 04.06.2013
(51) Int. Cl.: C07H 13/06, C07H 15/04, C07H 15/06, C07H 5/06, A61K 39/02, A61K 31/715, A61K 31/70, A61P 31/04, A61P 37/02

(54) **MODIFIED ENDOTOXIC BACTERIA LIPOPOLYSACCHARIDE (VARIANTS), COMBINATION OF MODIFIED LIPOPOLYSACCHARIDES (VARIANTS) AND, CONTAINING SAME, A VACCINE (VARIANTS) AND A PHARMACEUTICAL COMPOSITION (VARIANTS)**
MODIFIZIERTE LIPOPOLYSACCHARIDE AUS ENDOTOXISCHEN BAKTERIEN (VARIANTEN), KOMBINATION AUS MODIFIZIERTEN LIPOPOLYSACCHARIDEN (VARIANTEN) SOWIE IMPFSTOFF (VARIANTEN) UND PHARMAZEUTISCHE ZUSAMMENSETZUNG (VARIANTEN) DAMIT
LIPOPOLYSACCHARIDE MODIFIÉ DE BACTÉRIES ENDOTOXIQUES (VARIANTES), COMBINAISON DE LIPOPOLYSACCHARIDES MODIFIÉS (VRIANTES) ET VACCINS LES COMPRENANT (VARIANTES), ET COMPOSITION PHARMACEUTIQUE (VARIANTES)

(43) Date of publication of application: 13.04.2016
(73) Proprietor: Aparin, Petr Gennadievich, Moscow 121096 (RU); Lvov, Vyacheslav Leonidovich, Moscow, 125252 (RU); Elkina, Stanislava Ivanovna, Moscow 125284 (RU); Golovina, Marina Eduardovna, Moscow 115522 (RU)
(72) Inventor: LEDOV, Vladimir Alekseyevich, Moscowskaya obl. g. Klimovsk 142182 (RU); MARKINA, Anna Aleksandrovna, Moscow 111625 (RU); SHEKHT, Maria Evgen'evna, Moscow 125502 (RU)
(74) Representative: Leonard, Thomas Charles
(86) International application number: PCT/RU2013/000456
(87) International publication number: WO 2014/196887

(56) References cited:
- WO-A1-95/14026
- WO-A1-2012/154072
- RU-C2- 2 154 068
- US-A- 4 912 094
- US-A1- 2005 271 675
- YANYAN LI ET AL: "A rapid one-step method for the characterization of membrane lipid remodeling in Francisella using matrix-assisted laser desorption ionization time-of-flight tandem mass spectrometry", RAPID COMMUNICATIONS IN MASS SPECTROMETRY., vol. 25, no. 18, 22 August 2011 (2011-08-22), pages 2641-2648, XP055336001, GB ISSN: 0951-4198, DOI: 10.1002/rcm.5168
- YUKARI FUJIMOTO ET AL: "Synthesis of lipid A and its analogues for investigation of the structural basis for their bioactivity", JOURNAL OF ENDOTOXIN RESEARCH, vol. 11, no. 6, 1 December 2005 (2005-12-01), pages 341-347, XP055336126, UK ISSN: 0968-0519, DOI: 10.1179/096805105X76841
- U. OHTO ET AL: "Structural basis of species-specific endotoxin sensing by innate immune receptor TLR4/MD-2", PROCEEDINGS OF THE NATIONAL ACADEMY OF SCIENCES, vol. 109, no. 19, 8 May 2012 (2012-05-08), pages 7421-7426, XP055062412, ISSN: 0027-8424, DOI: 10.1073/pnas.1201193109
- SIMIN REZANIA ET AL: 'EXTRACTION PURIFICATION AND CHARACTERIZATION OF LIPOPOLYSACCHARIDE FROM ESCHERICHIA COLI AND SALMONELLA TYPHI' AVICENNA JOURNAL MED BIOTECH vol. 3, no. 1, 2011, pages 3 - 9, XP055284863

## Description

### Technical Field

The invention relates to the clinical immunology and pharmacology, in particular to modified lipopolysaccharides of endotoxic bacteria, specifically *Salmonella, Escherichia, Shigella, Bordetella, Haemophilus, Neisseria, Campylobacter, Vibrio, Klebsiella, Chlamydya, Corynobacterium* and their combinations, along with vaccines and pharmaceutical compositions comprising them.

### Prior Art

Lipopolysaccharides (LPS) are endotoxins of gram-negative bacteria and consist of polysaccharide (O-PS) and lipid components.

The lipid component, which is also referred to as lipid A, determines the endotoxic properties of lipopolysaccharides (Rietschel E.T., Kirikae T., Schade F.U., Ulmer A.J., Holst O., Brade H., Schmidt G., Mamat U., Grimmecke H.D., Kusumoto S. et al. The chemical structure of bacterial endotoxin in relation to bioactivity. Immunobiology, 1993, Apr; 187(3-5): 169-190). Polysaccharide component of LPS is a O-specific polysaccharide - O-PS composed of repeating oligosaccharide units and is connected to core oligosaccharide, which in turn is connected to lipid A. LPS composed of all three structural parts, O-PS, core and lipid A, are called S-LPS because they are characteristic of the «smooth» colonies of microorganisms. O-PS is absent in the LPS structure of some bacteria, including pathogenic bacteria. These microorganisms form rough colonies in many cases and produce low molecular LPS, so called R-LPS, which contain only core oligosaccharide linked to lipid A. It has been shown that the unique structure of O-PS determines the immunospecificity of S-LPS and of the whole microorganism as a consequence, and in many cases S-LPS-specific antibodies induced in the host in response to S-LPS play a key role against bacterial infection and exhibit high protective capacity.

However it is well known that, besides the ability to activate the adaptive immunity, LPS even in minimal doses are very endotoxic. In response to appearance of significant amounts of LPS in a macro-organism, there occurs a state of endotoxic shock, which rules out the use of LPS as components of vaccines and therapeutic drugs.

Endotoxic component of LPS - lipid A is usually disaccharide composed of two phosphorylated glucosamine residues, each of which is N- and O-acetylated (at the 3 and 3' positions) by four 3- hydroxymyristic acid residues (HMA) which are called primary. Two other non-hydroxylated higher fatty acid residues (typically lauric and myristic acids) O-acetylate two of the abovementioned HMA residues, which are called secondary. Therefore so called «classic» lipid A consists of six (four primary and two secondary) higher fatty acid residues (Knirel Yu.A., Valvano M. A. Bacterial Lipopolysaccharides. Structure, Chemical Synthesis, Biogenesis and Interaction with Host Cells. Springer Wien New York, 2011, 440 pp.).

It is believed that LPS of gram-negative microorganisms which does not contain «classic» lipid A with six higher fatty acid residues, but which contain smaller number of fatty acid residues in lipid A, have reduced level of endotoxicity.

It is believed that reduction of endotoxicity of S-LPS to clinically acceptable levels with retention of their ability to induce specific protective antibody to polysaccharide epitopes leads to principal possibility of using low endotoxic modified S-LPS of endotoxic bacteria, actual infectious agents, as immunoprophylactic vaccines and medicinal drugs.

The studies of transformations of endotoxic LPS containing «classic» lipid A to lower endotoxic derivatives prepared by methods of chemical, enzymatic and genetic detoxification have started over 20 years ago.

According to US Patent No. 4,912,094, penta-acetylated derivative of R-LPS (composed of lipid A with five fatty acid residues) of endotoxic bacteria - *Salmonella enterica sv minnesota* and *Escherichia coli,* were obtained by controlled alkaline hydrolysis of LPS resulting in selective cleavage of β-hydroxymyristic acid, which is connected to reducing end of glucosamine at the 3 position by ester bond. At the same time, data for the reduction of endotoxicity of modified R-LPS were very limited; it was noted that the toxicity decreased only about 50 times in the chicken embryo toxicity test. Pharmaceutical tests of toxicity reduction for R-LPS (side reactions and pyrogenicity tests) were not carried out.

According to US Patent No. 6,482,807, penta-acetylated LPS with reduced endotoxicity was obtained by genetic engineering from genetically modified recombinant *Neisseria meningitidis* bacteria by blocking production of hexa-acetylated form of LPS in cell. However thus obtained LPS demonstrated insignificant endotoxicity reduction (only 100-fold decrease of endotoxicity was observed according to the test data for the *in vitro* TNF-α production). The modified strain was only 7 times different from the initial strain by decreasing activity in LAL-test. Pharmaceutical pyrogenic assay data of LPS product were not presented. The product was described as an adjuvant, not a vaccine antigen.

It is clear at the present time that penta-acetylated LPS of endotoxic bacteria retain essential level of endotoxicity in experimental animals and thus they cannot find clinical application as components of pharmaceutical preparations.

According to US Patent No. 4,929,604, derivatives of lipid component of LPS of endotoxic bacteria, primarily composed of primary residues of 3-hydroxymyristic acid were obtained with acyloxyacyl hydrolase by the method of selective enzymatic hydrolysis. However, removal of secondary fatty acid was incomplete and only 80 - 90% of secondary fatty acids were removed even by using maximum time period of enzymatic treatment. Thus the product of this enzymatic treatment is a mixture of tetra-acylated derivatives of LPS (including lipid A with four fatty acid residues) with unreacted penta- and hexa-acylated derivatives of LPS (including lipid A with five and six fatty acid residues, respectively) of *Escherichia* or *Haemophilis* or *Neisseria.* Furthermore immunobiological properties of the mixture of tetraacyl derivatives with penta- and hexa-acylated derivatives, but not pure tetra-acetylated derivatives of LPS, were studied. The study results were unsatisfactory: only 20-fold decrease in activity of the mixture of modified LPS was observed in the LAL-test in comparison with the initial LPS. The reduction of level of pyrogenicity was insignificant (only 2.5 - 3 times) calculated by thermal response index in the rabbit pyrogenicity test. In the patent text the assessment of decrease in skin sensitization effect of maximum deacetylated LPS under study in Schwartzman reaction was described in a contradictory way (decreasing from 10- to 100-times).

The mixture of penta- and tetra-acetylated derivatives of R-LPS and S-LPS of endotoxic *H*. *influenzae* and *B. pertussis* bacteria was also prepared from genetically modified strains by transformation of their enzymatic systems (US 7,005,129 B1; WO 2006065139 A2).

However the obtained mixture of mutant LPS had high residual endotoxicity and cannot be used as potential immunogens for humans.

Thus the methods of enzymatic treatment described in patents leading to production of the mixture of tetra-, penta- and hexa-acylated derivatives of LPS of endotoxic bacteria do not result in significant endotoxicity reduction that prevents their clinical application as a component of pharmaceutical preparations.

Prior art shows that preparations of modified LPS of endotoxic bacteria which are pentaacylated derivatives or combination of tetra-, penta-, and hexaacetylated derivatives in no way meet clinical safety criteria. Apparently this is why authors of the abovementioned patents did not conduct standard studies of immunogenicity of preparations as candidate vaccines. The preparation of modified LPS was principally considered for use as immunostimulants and because of this reason the immunogenic study of the preparations and the evaluation of the immune response against O-antigen domain of LPS were not carried out.

Due to unsatisfactory safety level of abovementioned modified LPS, produced by inefficient deacetylation of lipid A, this line of research was practically abandoned. Therefore since the end of last century no patents have been obtained and no papers have been published dedicated to the preparation of other modified LPS and in particular modified S-LPS from actual endotoxic bacterial strains (genetically unmodified) and their application as a potential vaccines for administration to humans. The principal possibility of using di-, tri- and tetraacetylated derivatives of LPS of endotoxic bacteria as vaccines was not in view of the researchers. This area has never previously been considered as the subject of targeted practical application by vaccinologists.

The studies in the field of design of clinically applicable vaccines have moved to another paradigm, notably the use of cleavage elements of S-LPS - O-PS, fragment of O-PS-core with diglycosamine residues or lipid A obtained by full deacylation with hydrazine as specific antigen for conjugation with protein carriers. (US 7,553,490; Konadu E., Shiloach J., Bryla D.A., Robbins J.B., Szu S.C. Synthesis, characterization and immunological properties in mice of conjugates composed of detoxified lipopolysaccharide of Salmonella paratyphi A bound to tetanus toxoid with emphasis on the role of O-acetyls. Infect. Immun., 1996, July, 64(7): 2709-15; Pavliakova D., Moncrief J.S., Lyerly D.M., Schiffman G., Bryla D.A., Robbins J.B., Schneerson R. Clostridium difficile recombinant toxin A repeating units as a carrier protein for conjugate vaccines: studies of pneumococcal type 14, Escherichia coli K1 and Shigella flexneri type 2a polysaccharides in mice. Infect. Immun., 2000, Apr., 68(4):2161-6).

Therefore, the prior art does not suggest the claimed modified lipopolysaccharides, pure di-, tri-, and tetra-acetylated derivatives of LPS of endotoxic bacteria, and their clinical application as direct vaccine drug preparations, either as S-LPS individually or combinations thereof as two- and three-component active substances.

Information about deacylated LPS of *Porphyromonas gingivalis* bacteria - component of oral cavity microflora is indirectly relevant to the claimed objects. According to US. Patent No. 7,622,128, penta-, tetra-, tri-acetylated derivatives of *P. gingivalis* LPS were obtained and the principal possibility was demonstrated of using pentaacetylated and tetraacetylated derivatives as immunomodulators composed of the corresponding compositions. At the same time the data for characterizing their safety level and possibility of their clinical application are absent.

*P. gingivalis* produces LPS with fairly low endotoxicity and pyrogenicity (with 1000-fold decreasing ability to activate proinflammatory cytokines and with 100-fold decreasing toxicity in galactosamine model compared to LPS from endotoxic *E coli* bacteria), first of all it was explained by structural features of the lipid A.

*P. gingivalis* is agent of local low intensity infection of oral cavity and the use of its LPS as protective antigen against this infection requires development of special approaches in preventive vaccination. (Darveau R. P., Cunningham M.D., Bailey T., Seachord C., Ratcliffe K., Bainbridge B., Dietsch M., Page R.C., Aruffo A. Ability of bacteria associated with chronic inflammatory disease to stimulate E-selectin expression and promote neutrophil adhesion. Infect. Immun., 1995, Apr., 63(4):1311-7; Bainbridge B. and Darveau R. P. Porphyromonas gingivalis Lipopolysaccharide: an Unusual Pattern Recognition Receptor Ligand for the Innate Host Defense System. Acta. Odontol. Scand., 2001, 59:131-8).

It was shown that about half of fatty acids in LPS *P. gingivalis* lipid A had unusually branched structure and contains an odd number of carbon atoms, dramatically distinguishing this LPS from LPS of endotoxic bacteria containing «classic» lipid A.

The closest analogues of the claimed objects in the part of modified S-LPS of endotoxic bacteria are solely for formal reasons the technical solutions of US Patent No. 4,912,094 and WO 9514026 A1.

In unreasonably broad patent claims of these patents, the general structural formula is characterized by a large group of modified S-LPS, R-LPS and modified lipid A of endotoxic bacteria, while only penta-acetylated derivatives of R-LPS and lipids A of *S. enterica sv minnesota* and *E. coli* in the former case and tri-acetylated and tetra-acetylated derivatives of *E. coli, Haemophilis influenzae* and *Pseudomonas aeruginosa* lipid A in the latter case, was practically obtained and studied for immunobiological properties.

The data in US Patent No. 4,912,094 were discussed above, while information in WO 9514026 A1 is subject to discussion in details.

Tri-acetylated derivatives of lipid A of abovementioned bacteria were powerful inducers of a key mediator of endotoxin reaction - TNF-α *in vivo,* and also of another pro-inflammatory cytokine - IL-6 (data from analogue patent RU 2154068 C2). In the culture of PMN *in vitro* tri-acylated derivatives of lipid A induced even higher TNF-α production in comparison with LPS Westphal.

In this regard, the conclusions about low endotoxicity of preparations of modified lipid A based on its low activity in LAL- test (1000-fold decreasing) in the document WO 9514026 A1 and in the analogue patents are unfounded. The evaluation of endotoxicity of LPS is incomplete only on the basis of *in vitro* LAL-test based on gel-forming activity of Limulus protein, ignoring the test results *in vivo,* reflecting production of pro-inflammatory cytokines (plasma concentration, pyrogenicity, side reactions). There are no data on pyrogenicity level and thus on the safety of obtained products.

Subsequent author publications of indicated documents go to prove that tri- and tetra-acylated lipid A developed as component of anticancer immune drugs, having essential residual endotoxicity and safety level, are not acceptable as traditional vaccine drugs (Brandenburg K., Lindner B., Schromm A., Koch M.H.J., Bauer J., Merkli A., Zbaeren C., Davies J.G., Seydel U. Physicochemical characteristics of triacyl lipid A partial structure OM-174 in relation to biological activity. Eur. J. Biochem., 2000, v. 267, pp. 3370-7). Modified lipid A cannot be used as a vaccine (it does not contain O-PS bacteria antigen) and this is a problem in terms of using them as additional component - adjuvant because of absence of data supporting their low pyrogenicity.

In the light of the foregoing, there were absolutely unexpected results of the comparative study of the induction *in vivo* of pro-inflammatory cytokine and mediator of endotoxin reaction - TNF-α by tri-acetylated lipid A from *E.coli* OM-174 and thus di-, tri-, and tetraacetylated endotoxic bacteria S-LPS, respectively, conducted by authors of the present application and described in Example 1.

In distinction to modified lipid A, corresponding modified S-LPS of endotoxic bacteria were poor inducers of endotoxic shock and exhibits low pyrogenicity and endotoxicity for laboratory animal and human subjects.

These original results introduce certain corrections in the generally accepted view of contribution of the polysaccharide component to immunobiological properties of S-LPS, and also allow to establish correlation between the extent of modification of LPS of endotoxic bacteria and optimal ratio of their safety and immunogenicity levels, which opens the perspectives of their clinical use.

Fujimoto et al. (2003) describes the structural requirements for the endotoxic and antagonistic activity of lipid A derivatives.

Ohto et al. (2012) describes structural evidence of the agonistic property of lipid IVa on mouse TLR4/MD-2.

US 4,912,094 provides modified lipopolysaccharides that are less endotoxic and maintain their antigenic and immune-stimulating properties.

Li et al. (2011) describes a one-step protocol for the combined isolation of phospholipids and lipid A from *Francisella* subspecies followed by analysis using matrix-assisted laser desorption ionization time-of-flight tandem mass spectrometry. This analysis showed that the bacterial membranes remodeled rapidly to adapt to changes in environmental growth conditions and may be important for *Francisella* pathogenesis.

### Disclosure of the Invention

The subject matter for which protection is sought is as defined by the claims.

The present invention provides a modified lipopolysaccharide (S-LPS) of endotoxic bacteria, wherein said modified lipopolysaccharide is free of lipopolysaccharides containing lipid A having five acyl residues or six acyl residues, comprising:
a) O-specific polysaccharide antigen consisting of one or more repeating units;
b) core oligosaccharide; and
c) either
   (i) fully O-deacylated lipid A containing two acyl residues; or
   (ii) partially O-deacylated lipid A containing three or four acyl residues.

The present invention also provides a combination of the modified lipopolysaccharides, wherein said modified lipopolysaccharide is free of lipopolysaccharides containing lipid A having five acyl residues or six acyl residues, that comprises (a) a modified lipopolysaccharide (S-LPS) of endotoxic bacteria comprising: O-specific polysaccharide antigen consisting of one or more repeating units, core oligosaccharide and fully O-deacylated lipid A containing two acyl residues, and (b) a modified lipopolysaccharide (S-LPS) of endotoxic bacteria comprising: O-specific polysaccharide antigen consisting of one or more repeating units, core oligosaccharide and partially O-deacylated lipid A containing three acyl residues.

The present invention also provides a combination of the modified lipopolysaccharides, wherein said modified lipopolysaccharide is free of lipopolysaccharides containing lipid A having five acyl residues or six acyl residues, that comprises (a) a modified lipopolysaccharide (S-LPS) of endotoxic bacteria comprising: O-specific polysaccharide antigen consisting of one or more repeating units, core oligosaccharide and fully O-deacylated lipid A containing two acyl residues, (b) a modified lipopolysaccharide (S-LPS) of endotoxic bacteria comprising: O-specific polysaccharide antigen consisting of one or more repeating units, core oligosaccharide and partially O-deacylated lipid A containing three acyl residues, and (c) a modified lipopolysaccharide (S-LPS) of endotoxic bacteria that comprises: O-specific polysaccharide antigen consisting of one or more repeating units, core oligosaccharide and partially O-deacylated lipid A containing four acyl residues.

The present invention also provides a combination of modified lipopolysaccharides, wherein said modified lipopolysaccharide is free of lipopolysaccharides containing lipid A having five acyl residues or six acyl residues, comprising (a) a modified lipopolysaccharide (S-LPS) of endotoxic bacteria, comprising: O-specific polysaccharide antigen consisting of one or more repeating units, core oligosaccharide and fully O-deacylated lipid A containing two acyl residues, and (b) a modified lipopolysaccharide (S-LPS) of endotoxic bacteria, comprising: O-specific polysaccharide antigen consisting of one or more repeating units, core oligosaccharide and partially O-deacylated lipid A containing four acyl residues.

The present invention also provides a combination of the modified lipopolysaccharides, wherein said modified lipopolysaccharide is free of lipopolysaccharides containing lipid A having five acyl residues or six acyl residues, that comprises (a) a modified lipopolysaccharide (S-LPS) of endotoxic bacteria, comprising: O-specific polysaccharide antigen consisting of one or more repeating units, core oligosaccharide and partially O-deacylated lipid A containing three acyl residues, and (b) a modified lipopolysaccharide (S-LPS) of endotoxic bacteria, comprising: O-specific polysaccharide antigen consisting of one or more repeating units, core oligosaccharide and partially O-deacylated lipid A containing four acyl residues.

The present invention also provides composition containing the modified lipopolysaccharide (S-LPS) of endotoxic bacteria of the invention or the combination of modified lipopolysaccharides of the invention for use in the treatment or prophylaxis of septic shock or endotoxic shock or in the treatment of influenza or for use as a therapeutic or preventative vaccine for bacterial or viral infection.

The present invention also provides a vaccine comprising the modified lipopolysaccharide (S-LPS) of endotoxic bacteria of the invention or the combination of modified lipopolysaccharides of the invention.

The invention also provides a pharmaceutical composition comprising the modified lipopolysaccharide (S-LPS) of endotoxic bacteria of the invention or the combination of modified lipopolysaccharides of the invention.

The objectives of the claimed inventions are:
(i) preparation of pure individual (free from impurities of penta- and hexaacylated derivatives) modified S-LPS of endotoxic bacteria (di-, tri- and tetra-acylated derivatives) and combinations thereof;
(ii) development of vaccines and pharmaceutical compositions on the basis of indicated objects containing modified S-LPS as individual monocomponent or combinations thereof as two- and three-component active substance, respectively.
The technical results, provided by the claimed inventions, are:
(a) high safety level (there are no endotoxin reactions - chills, fever, tachycardia, increase in arterial blood pressure when individual modified S-LPS or combinations thereof were parenterally administered to volunteers in a single dose of up to 200 mcg);
(b) low pyrogenicity (1,000 - 10,000-fold decrease of pyrogenic dose of individual modified S-LPS and combinations thereof in the rabbit pyrogenicity test in comparison with classic LPS Westphal; slight, up to 37.6 °C temperature reaction when indicated products were parenterally administered to volunteers in a single dose of up to 200 mcg);
(c) anti-shock activity (pre-administration of individual modified S-LPS or combinations thereof provides 80% animal survival rate undergoing endotoxic shock induced by administration of lethal dose of endotoxin; moreover administration of indicated products provides the correction of septic shock and slows down the development of peritonitis for 18-30 hours);
(d) high efficiency and specificity (immunization of volunteers and experimental animals with individual modified S-LPS or combinations thereof determines the production of high levels of LPS-specific and O-antigen specific IgG, IgA, IgM; four-fold sero-conversion of antibodies and direct animal protection in experimental infection models);
(e) broad-spectrum action (development of combined multivalent vaccines containing individual modified S-LPS or their combinations on the basis of two or several serotypes);
(f) synergistic effect observed for the combinations of modified S-LPS with regard to pyrogenicity reduction and immunogenicity enhancement;
(g) good chemical-pharmaceutical characteristics of individual modified S-LPS as well as their combinations (thermostability, extended storage period, environmental resistance).

For the first time, individual (free from impurities of penta- and hexaacylated derivatives) di-, tri- and tetraacylated S-LPS of endotoxic bacteria and also combinations thereof were obtained and their immunobiological, physical-chemical, chemical-pharmaceutical properties were investigated.

To clarify the scope of claims with regard to sources of claimed objects we should define a notion of «endotoxic bacteria» of *Salmonella, Escherichia, Shigella, Bordetella,* Haemophilus, *Neisseria, Campylobacter, Vibrio, Klebsiella, Chlamydya, Corynobacterium* genera.

Such bacteria should be called strains of natural genetically unmodified, gram-negative bacteria obtained from patients with infectious diseases or other environmental sources which produce endotoxic agonistic form of LPS molecules, which are characterized by high toxicity and pyrogenicity in free or associated with cell forms and high degree of acylation of lipid A (penta- or hexaacylated). The course of infectious process induced by endotoxic strain of bacteria -active producer of endotoxic LPS, is burdened by apparent temperature reactions, fever and other signs of Systemic Inflammatory Response Syndrome (SIRS).

On the other hand, low-endotoxic, gram-negative, naturally occurring bacteria have 100 - 1000 times lower endotoxicity. So it requires 100 - 1000 times more cells of low-endotoxic bacteria (*Helicobacter pylori, P.gingivalis, Treponema pallidum*) compared with amount of endotoxic bacteria cell (*E. coli*) to achieve the similar level of activation of epithelial cells receptors, PMN, TNF- α, IL-6. (Darveau R. P.,Cunningham M.D., Bailey T., Seachord C., Ratcliffe K., Bainbridge B., Dietsch M., Page R.C., Aruffo A. Ability of bacteria associated with chronic inflammatory disease to stimulate E-selectin expression and promote neutrophil adhesion. Infect. Immun., 1995, Apr., 63(4):1311-7).

Low-endotoxic bacteria are either virulent free or they cause low intensity, often subclinical forms of mucosal infections, that is why they are often called "invisible bacteria". LPS of low-endotoxic bacteria always has structural features which are responsible for their low endotoxicity - low degree of lipid A acylation (tri- or tetraacylation), dephosphorylated form of lipid A, unique fatty acid structure (Alexander C., Rietschel E.T. Bacterial lipopolysaccharides and innate immunity. J. Endotoxin Res., 2001, v.7, pp. 167-202.). Modified lipopolysaccharide (S-LPS) of endotoxic bacteria was obtained comprising: Ospecific polysaccharide, consisting of one or more repeating units, core oligosaccharide and fully O-deacylated lipid A with two acyl residues.

The claimed lipopolysaccharide has no less than 85% purity (Example 2B), generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Examples 2D, 3F), has anti-shock activity for septic and/or endotoxic shock and is the immune system response modulator in mammals, including humans (Examples 3F, 4C), and is apyrogenic for a rabbit in a dose of up to 100 mcg/kg in the rabbit pyrogenicity test (Example 2C).

Modified lipopolysaccharide (S-LPS) of endotoxic bacteria was obtained comprising: O-specific polysaccharide, consisting of one or more repeating units, core oligosaccharide and partially O-deacylated lipid A with three acyl residues.

The claimed lipopolysaccharide has no less than 80% purity (Example 2B), generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Examples 2D, 3C, 3F), has anti-shock activity for septic and/or endotoxic shock (Examples 3D) and is the immune system response modulator in mammals, including humans, and is apyrogenic for a rabbit in a dose of up to 100 mcg/kg in the rabbit pyrogenicity test (Example 2C).

Modified lipopolysaccharide (S-LPS) of endotoxic bacteria was obtained comprising: O-specific polysaccharide consisting of one or more repeating units, core oligosaccharide, and partially O-deacylated lipid A with four acyl residues.

The claimed lipopolysaccharide has no less than 80% purity (Example 2B), generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Example 2D), has anti-shock activity for septic and/or endotoxic shock and is the immune system response modulator in mammals, including humans, and is apyrogenic for a rabbit in a dose of up to 100 mcg/kg in the rabbit pyrogenicity test (Example 2C).

The combination of modified lipopolysaccharides (S-LPS) of endotoxic bacteria was obtained comprising diacylated and triacylated derivatives at any ratio. The claimed combination exhibits synergistic effect with regard to immunogenicity enhancement compared with diacylated lipopolysaccharide derivative (Example 2D), generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Example 2D), has anti-shock activity for septic and/or endotoxic shock and is the immune system response modulator in mammals, including humans (Examples 3C), and is apyrogenic for a rabbit in a dose of up to 100 mcg/kg in the rabbit pyrogenicity test (Example 2C).

The combination of modified lipopolysaccharides (S-LPS) of endotoxic bacteria was obtained comprising diacylated and tetraacylated derivatives at any ratio. The claimed combination exhibits synergistic effect with regard to immunogenicity enhancement compared with di-acylated lipopolysaccharide derivative (Example 2D), generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Example 2D), has anti-shock activity for septic and/or endotoxic shock and is the immune system response modulator in mammals, including humans (Examples 3C), and is apyrogenic for a rabbit in a dose of up to 100 mcg/kg in the rabbit pyrogenicity test (Example 2C).

The combination of modified lipopolysaccharides (S-LPS) of endotoxic bacteria was obtained comprising tri-acylated and tetra-acylated derivatives at any ratio. The claimed combination exhibits synergistic effect with regard to immunogenicity enhancement compared with tetra-acylated lipopolysaccharide derivative, generates protection against *Salmonella, Escherichia, Shigella, Bordetella, Haemophilus, Neisseria,* Campylobacter, Vibrio, *Klebsiella, Chlamydya, Corynobacterium* and combinations thereof by inducing a synthesis of specific antibacterial IgG, IgA, IgM antibodies in mammals, including humans (Example 2D), has anti-shock activity for septic and/or endotoxic shock and is the immune system response modulator in mammals, including humans, and is apyrogenic for a rabbit in a dose of up to 100 mcg/kg in the rabbit pyrogenicity test (Example 2C).

The combination of modified lipopolysaccharides (S-LPS) of endotoxic bacteria was obtained comprising di-acylated, tri-acylated and tetra-acylated derivatives at any ratio. The claimed combination exhibits synergistic effect with regard to immunogenicity enhancement compared with di-acyled and tri-acylated lipopolysaccharide derivatives (Example 2D, 3F), generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Examples, 2D, 3F, 3C), has antishock activity for septic and/or endotoxic shock (Example 3D) and is the immune system response modulator in mammals, including humans (Examples 3F, 4B, 4C), and is apyrogenic for a rabbit in a dose of up to 100 mcg/kg in the rabbit pyrogenicity test (Example 2C).

Vaccine was developed for prophylaxis and/or treatment of infectious diseases caused by gram-negative bacteria.

The claimed vaccine comprises the preventive and/or therapeutically effective amount of the modified lipopolysaccharide (S-LPS) of endotoxic bacteria - its di-acylated or tri-acylated, or tetra-acylated derivative. The claimed vaccine generates protection against *Salmonella, Escherichia, Shigella, Bordetella, Haemophilus, Neisseria,* Campylobacter, Vibrio, *Klebsiella, Chlamydya, Corynobacterium* and combinations thereof by inducing a synthesis of specific antibacterial IgG, IgA, IgM antibodies in mammals, including humans (Examples 3C, 3F), and provides prophylaxis and/or correction of the course of septic and/or endotoxic shock (Example 3D). In the claimed vaccine the modified lipopolysaccharides are apyrogenic for a rabbit in a dose of up to 100 mcg/kg in the rabbit pyrogenicity test (Example 3B).

The claimed vaccine may comprise pharmaceutically acceptable additives, which may be pH stabilizers or preservatives, or adjuvants, or isotonizing agents, or combinations thereof (Example 3A). Moreover, the vaccine may comprise modified lipopolysaccharide in nonconjugated as well as in conjugated form. Meanwhile, the vaccine, comprised of the conjugated form of the lipopolysaccharide, additionally contains carrier protein, namely diphtheria toxoid or tetanus toxoid, or *P.aeruginosa* exoprotein A, or other proteins (Example 3H, 3I).

The claimed vaccine comprises the preventive and/or therapeutically effective amount of the combination of the modified lipopolysaccharides (S-LPS) of endotoxic bacteria - di-acylated and tri-acylated derivatives or di-acylated and tetra-acylated derivatives, or tri-acylated and tetra-acylated derivatives, or di-acylated, tri-acylated and tetra-acylated derivatives. The claimed vaccine generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Examples 3C, 3F), and provides prophylaxis and/or correction of the course of septic and/or endotoxic shock (Example 3D). In the claimed vaccine combinations of the modified lipolysaccharides are apyrogenic for a rabbit in a dose of up to 100 mcg/kg in the rabbit pyrogenicity test (Example 3B).

The claimed vaccine may comprise pharmaceutically acceptable additives, which may be pH stabilizers or preservatives, or adjuvants, or isotonizing agents, or combinations thereof (Example 3A). Moreover, the vaccine may comprise modified lipopolysaccharide in nonconjugated as well as in conjugated form. Meanwhile, the vaccine, comprised of the conjugated form of the lipopolysaccharide, additionally contains carrier protein, namely diphtheria toxoid or tetanus toxoid, or *P.aeruginosa* exoprotein A, or other proteins (Example 3G).

It should be noted that the claimed vaccines on the basis of individual S-LPS and combinations thereof can simultaneously induce the broadest spectrum of antibodies to various antigen determinants of different domains of LPS molecule (O-PS, outer core, inner core), that can be considered as an important factor of an efficiency of protective immunity. In addition, the possibility of development of multivalent vaccine have been demonstrated in which each of monovaccine variants contain an antigen specific to the most epidemically significant strain of gram-negative bacteria (Example 3I).

The pharmaceutical composition was developed comprising the effective amount of the modified lipopolysaccharide (S-LPS) of endotoxic bacteria - its di-acylated or tri-acylated, or tetra-acylated derivative. The claimed pharmaceutical composition is the immune system response modulator in mammals, including humans (Example 4C); the modified lipopolysaccharide containing in its formulation is apyrogenic for a rabbit in a dose of up to 100 mcg/kg in the rabbit pyrogenicity test (Example 2C).

The claimed pharmaceutical composition may comprise pharmaceutically acceptable additives, which may be preservatives or stabilizers, or solvents, or combinations thereof. The claimed pharmaceutical composition has a broad-spectrum pharmacological activity and notably exhibits effective therapeutic antiviral action against infection caused by influenza A H1N1 virus (Example 4B). Moreover the pharmaceutical composition exhibits tolerogenic effect for the correction of pathological conditions, associated with increased production of proinflammatory cytokines (Example 4C).

The pharmaceutical composition was developed comprising the effective amount of the combination of the modified lipopolysaccharides (S-LPS) of endotoxic bacteria - di-acylated and tri-acylated derivatives or di-acylated and tetra-acylated derivatives, or tri-acylated and tetra-acylated derivatives, or di-acylated, tri-acylated and tetra-acylated derivatives. The claimed pharmaceutical composition is the immune system response modulator in mammals, including humans (Example 4B, 4C); combinations of the modified lipopolysaccharides containing in its formulation are apyrogenic for a rabbit in a dose of up to 100 mcg/kg in the rabbit pyrogenicity test (Example 2C).

The claimed pharmaceutical composition may comprise pharmaceutically acceptable targeted additives, which may be preservatives or stabilizers, or solvents, or combinations thereof. The claimed pharmaceutical composition has broad-spectrum pharmacological activity and notably exhibits effective therapeutic antiviral action against infection caused by influenza A H1N1 virus (Example 4B). Moreover the pharmaceutical composition exhibits tolerogenic effect for the correction of pathological conditions, associated with increased production of proinflammatory cytokines (Example 4C).

It should also be noted that undoubted advantage of the claimed medicaments (vaccine and pharmacompositions) in comparison with medicaments-analogues of other classes is their excellent chemical and pharmaceutical characteristics: thermostability, well known for LPS molecules, providing the possibility to its extended storage period, relative environmental resistance.

A use of the modified lipopolysaccharide (S-LPS) of endotoxic bacteria - its di-acylated or tri-acylated, or tetra-acylated derivative is also disclosed in the manufacture of a medicament. Meanwhile the modified lipopolysaccharide generates protection against *Salmonella, Escherichia, Shigella, Bordetella, Haemophilus, Neisseria, Campylobacter, Vibrio, Klebsiella, Chlamydya, Corynobacterium* and combinations thereof by inducing a synthesis of specific antibacterial IgG, IgA, IgM antibodies in mammals, including humans (Examples 2D, 3C), provides prophylaxis and/or correction of the course of septic and endotoxic shock (Example 3D), is the immune system response modulator in mammals, including humans (Example 4C); and is apyrogenic for a rabbit in a dose of up to 100 mcg/kg in the rabbit pyrogenicity test (Example 2B).

The medicament is intended for parenteral, peroral, rectal, intra-vaginal, transdermal, sublingual and aerosol administration to mammals, including humans.

The use of the combinations of the modified lipopolysaccharides (S-LPS) of endotoxic bacteria - di-acylated and tri-acylated derivatives or di-acylated and tetra-acylated derivatives, or tri-acylated and tetra-acylated derivatives, or di-acylated, tri-acylated and tetra-acylated derivatives is disclosed in the manufacture of a medicament.

Meanwhile the combinations of the modified polysaccharides generate protection against *Salmonella, Escherichia, Shigella, Bordetella, Haemophilus, Neisseria, Campylobacter, Vibrio, Klebsiella, Chlamydya, Corynobacterium* and combinations of them by inducing a synthesis of specific antibacterial IgG, IgA, IgM antibodies in mammals, including humans (Examples 2D, 3F), provide anti-shock activity for septic and endotoxic shock (Example 3D), are the immune system response modulators in mammals, including humans (Examples 3F, 4B, 4C); and are apyrogenic for a rabbit in a dose of up to 100 mcg/kg in the rabbit pyrogenicity test (Example 2C).

The medicament is intended for parenteral, peroral, rectal, intra-vaginal, transdermal, sublingual and aerosol administration to mammals, including humans.

The use of the modified lipopolysaccharide (S-LPS) - its di-acylated or tri-acylated, or tetraacylated derivative is claimed as an immunostimulating carrier in the manufacture of a vaccine against bacterial, viral and other infections.

Meanwhile the modified lipopolysaccharide is conjugated with protective antigen or hapten, which preferably has synthetic or protein, or polysaccharide nature.

The modified polysaccharide is apyrogenic for a rabbit in a dose of up to 100 mcg/kg in the rabbit pyrogenicity test (Example 2C).

The use of the combinations of modified lipopolysaccharides (S-LPS) - di-acylated and tri-acylated derivatives or di-acylated and tetra-acylated derivatives, or tri-acylated and tetra-acylated derivatives, or di-acylated, tri-acylated and tetra-acylated derivatives is claimed as an immunostimulating carrier in the manufacture of a vaccine against bacterial, viral and other infections.

Meanwhile the modified lipopolysaccharides are conjugated with protective antigen or hapten, which preferably have synthetic or protein, or polysaccharide nature.

The combinations of the modified lipopolysaccharides are apyrogenic for a rabbit in a dose of up to 100 mcg/kg in the rabbit pyrogenicity test (Example 2C).

### Brief Description of the Drawings

The inventions are illustrated by the following figures.

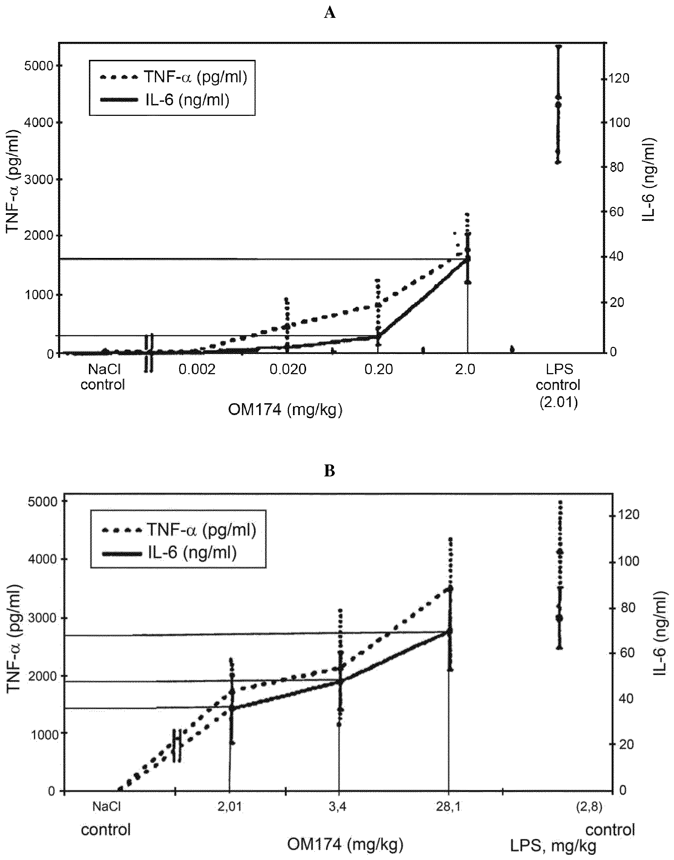

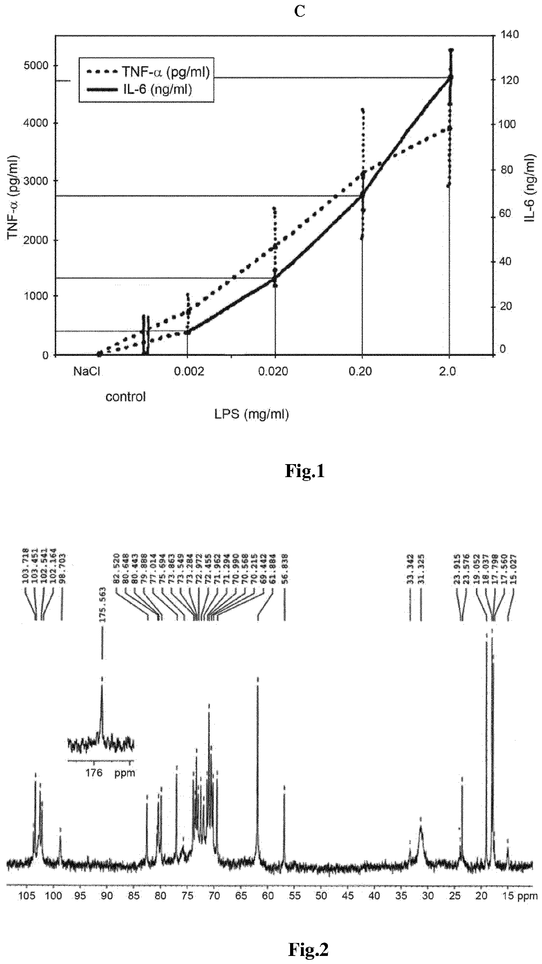

Fig.1 shows graphs of *in vivo* production of TNF- α the mediator of endotoxin reaction and proinflammatory cytokine IL-6 in blood sera after intravenous administration to mice of tri-acylated lipid A *E.coli* OM174 (A and B) and Westphal LPS *E coli* O:111B4 (C) based on data from patent RU 2154068. In this case the vertical axis represents the values for TNF- α concentrations (pg/mL) and the values for IL-6 concentrations (ng/mL). The horizontal axis represents (A and B) the values for injection dose of preparation OM174 (mg/kg) to mice. The vertical axis (C) represents the value for injection dose of LPS (mg/mL; positive control) and saline solution (0.85% NaCl solution; negative control) to mice.
   The solid lines represent the lines of extrapolation for the values of doses for OM174: 0.20; 2.00; 2.01; 3.40 and 28.10 (A and B) and the values of doses for Westphal LPS *E coli* O:111B4: 0.002; 0.020; 0.200 and 2.000 (C).
Fig.2 shows 13C-NMR-spectrum of *S. flexneri* 2a deacylated S-LPS.

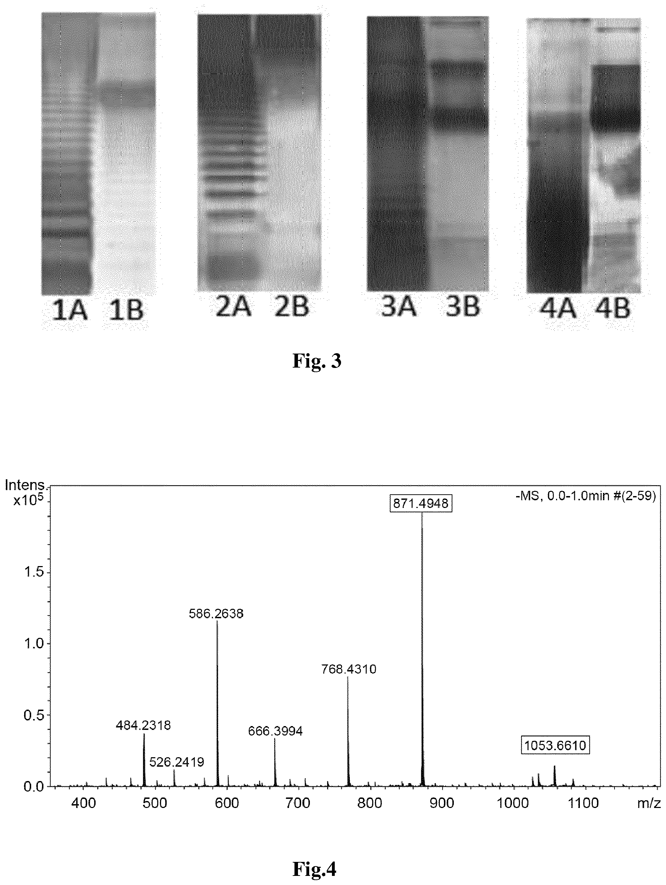

Fig.3 shows the photographs of silver staining tracks obtained after SDS-PAGE for the samples of the original LPS Westphal (A) and the modified S-LPS (B) *S.flexneri* 2a (1A, 1B), *E. coli* O:55 (2A, 2B), *K.pneumoniae* (3A, 3B), *S.enterica sv typhi* O:901 (4A, 4B).
Fig.4 shows ESI-MS mass-spectrum of lipid A obtained from *S.flexneri* 2a di-acylated S-LPS.

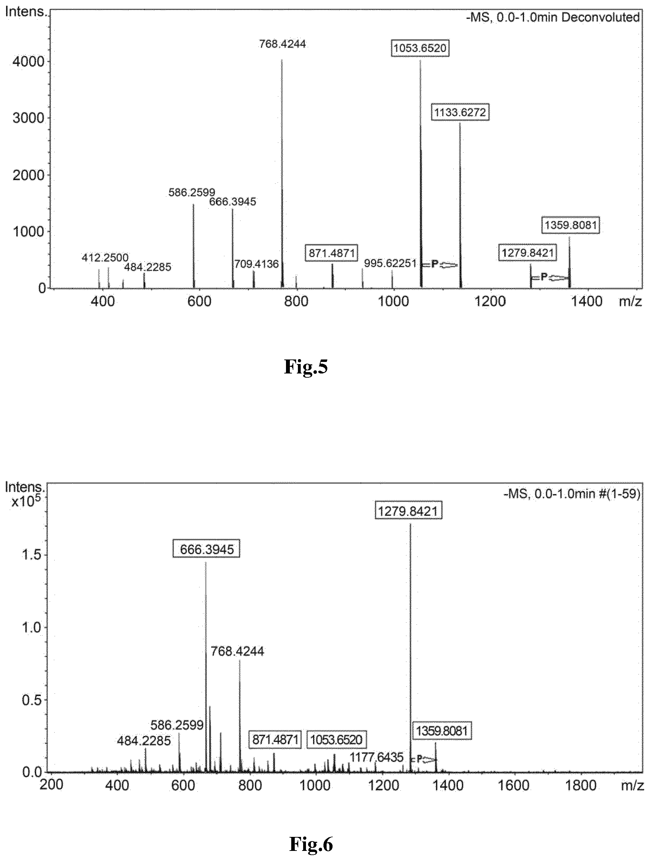

Fig.5 shows ESI-MS mass-spectrum of lipid A obtained from *S.flexneri* 2a tri-acylated S-LPS.
Fig.6 shows ESI-MS mass-spectrum of lipid A obtained from *S.flexneri* 2a tetra-acylated SLPS.

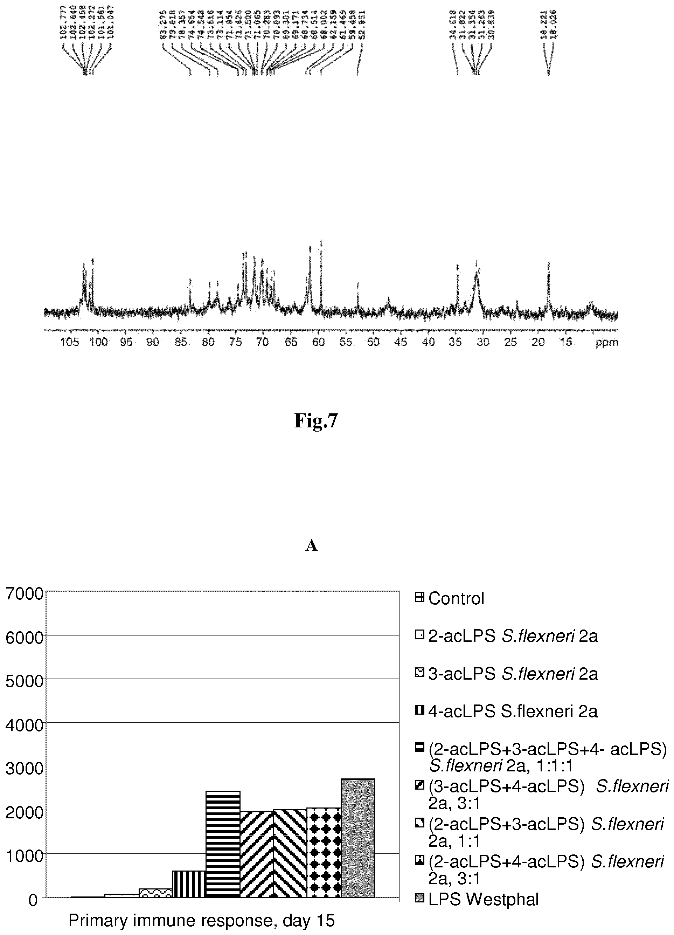

Fig.7 shows 13C-NMR-spectrum of pre-deacylated *S. enterica sv typhi* O: 901 S-LPS.

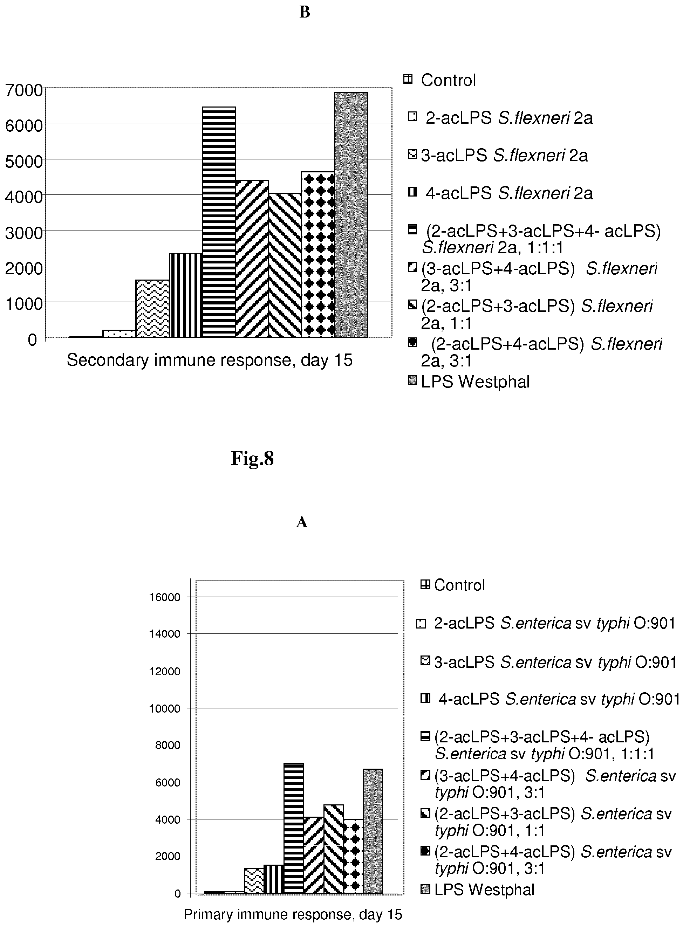

Fig.8 shows the diagrams of IgG antibody production (day 15) after primary (A) and secondary (B) immunization of mice with preparations made with 2-acLPS, 3-acLPS 4-acLPS *S. flexneri* 2a, and also combinations thereof (2-acLPS + 3-acLPS + 4-acLPS), (3-acLPS + 4-acLPS), (2-acLPS + 3-acLPS) and (2-acLPS + 4-acLPS) with component mass ratio 1:1:1, 3:1, 1:1 and 3:1, respectively, and the preparation made with Westphal LPS *S*. *flexneri* 2a, at a dose of 25 mcg per mouse. The vertical axis represents the value of titer serum dilution.

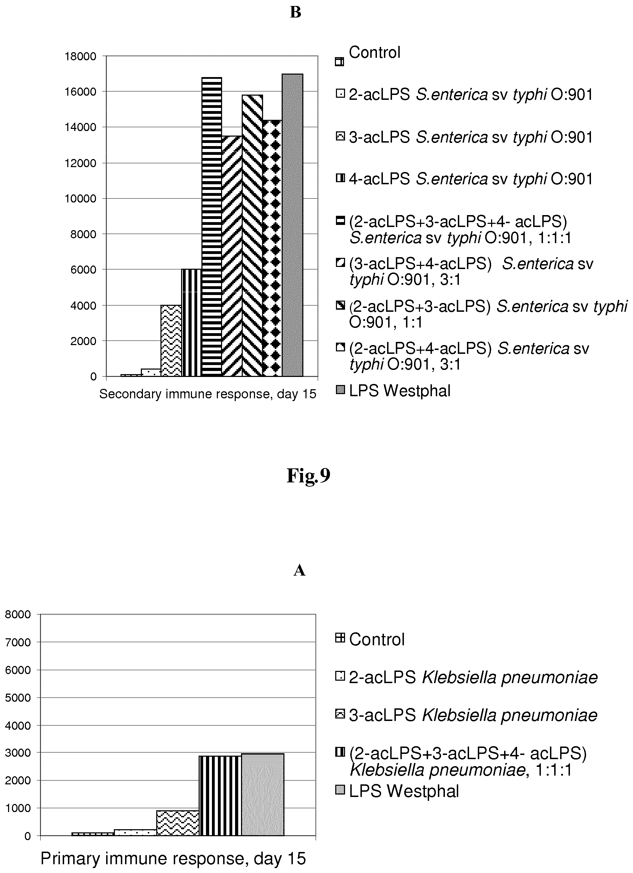

Fig.9 shows the diagrams of IgG antibody production (day 15) after primary (A) and secondary (B) immunization of mice with preparations made with 2-acLPS, 3-acLPS 4-acLPS *S.enterica sv typhi* O:901, and also combinations thereof (2-acLPS + 3-acLPS + 4-acLPS), (3-acLPS + 4-acLPS), (2-acLPS + 3-acLPS) and (2-acLPS + 4-acLPS) with component mass ratio 1:1:1, 3:1, 1:1 and 3:1, respectively, and the preparation made with Westphal LPS *S.enterica sv typhi* O:901, at a dose of 25 mcg per mouse. The vertical axis represents the value of titer serum dilution.

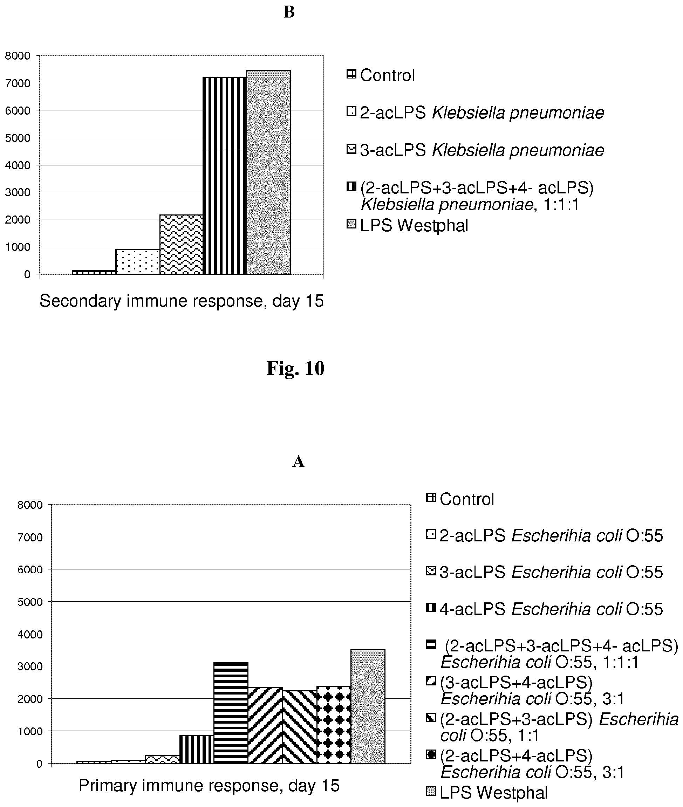

Fig.10 shows the diagrams of IgG antibody production (day 15) after primary (A) and secondary (B) immunization of mice with preparations made with 2-acLPS and 3-acLPS *K.pneumoniae,* and also combinations thereof (2-acLPS + 3-acLPS + 4-acLPS) with component mass ratio 1:1:1, and preparation made with Westphal LPS *K.pneumoniae,* at a dose of 25 mcg per mouse. The vertical axis represents the value of titer serum dilution.

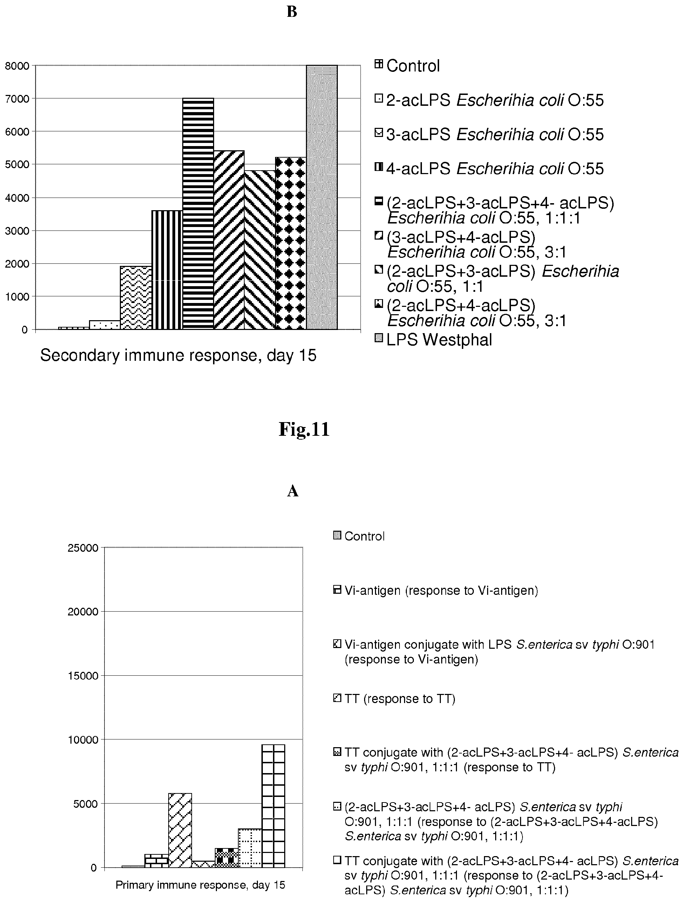

Fig.11 shows the diagrams of IgG antibody production (day 15) after primary (A) and secondary (B) immunization of mice with preparations made with 2-acLPS, 3-acLPS and 4-acLPS *E.coli* O:55, and also combinations thereof (2-acLPS + 3-acLPS + 4-acLPS), (3-acLPS + 4-acLPS), (2-acLPS + 3-acLPS) and (2-acLPS + 4-acLPS) with component mass ratio 1:1:1, 3:1, 1:1 3:1, respectively, and the preparation made with Westphal LPS *E.coli* O:55, at a dose of 25 mcg per mouse. The vertical axis represents the value of titer serum dilution.

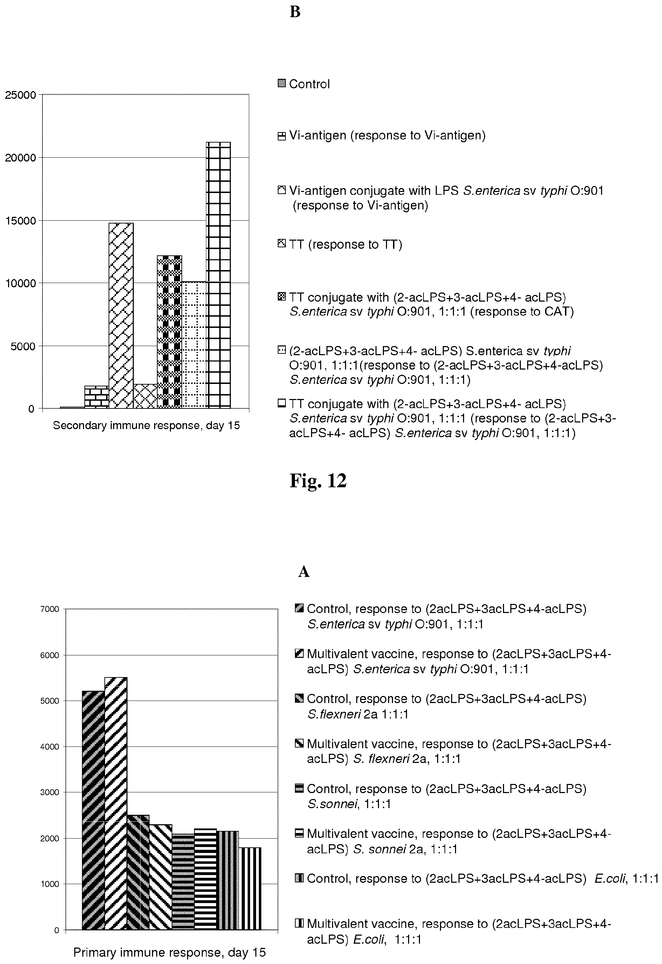

Fig.12 shows the diagrams of IgG antibody production (day 15) after primary (A) and secondary (B) immunization of mice with conjugations made with Vi-antigen and Tetanus Toxoid (TT) with immunostimulating carrier - the combination of (2-acLPS + 3-acLPS + 4-acLPS) *S.enterica sv typhi* O:901 with component mass ratio 1:1:1, and also pure Vi-antigen and TT, at a dose of 25 mcg of polysaccharide or 20 mcg of protein per mouse. The vertical axis represents the value of titer serum dilution.

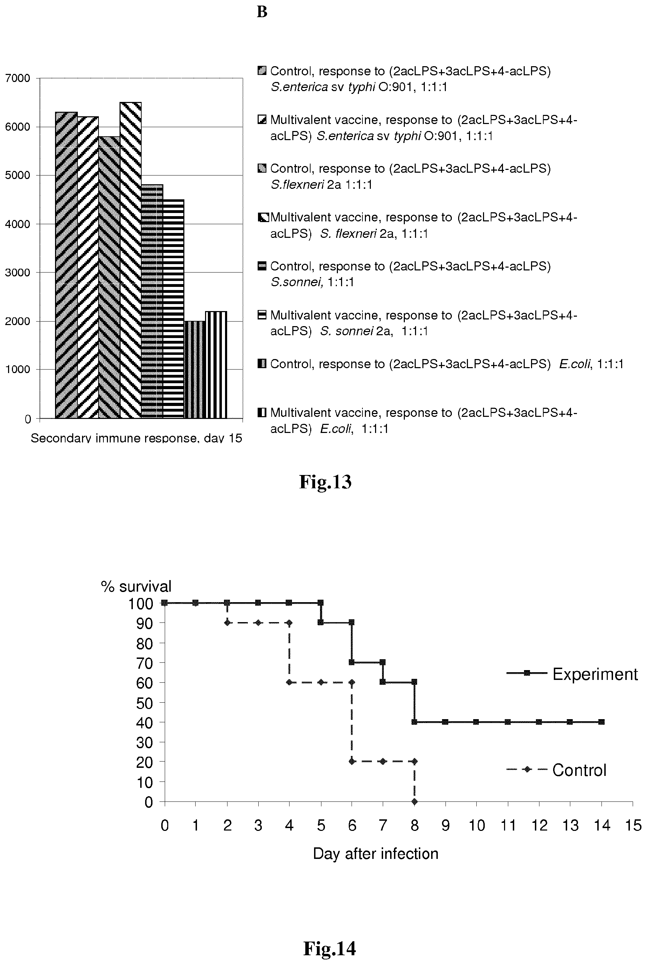

Fig.13 shows the diagrams of IgG antibody production (day 15) after primary (A) and secondary (B) immunization of mice with multivalent dysentery-typhoid-escherichia vaccine at a dose of 100 mcg per mouse, and also individual components thereof - combination of (2-acLPS + 3-acLPS + 4-acLPS) in mass ratio 1:1:1, *S.flexneri* 2a or *S.sonnei,* or *S.enterica sv typhi* O:901, or *E.coli* O:55, at a dose of 25 mcg per mouse. The vertical axis represents the value of titer serum dilution.
Fig.14 shows graphs of survival rates of two groups of mice, infected with a dose of LD100 of virulent influenza strain A subtype H1N1.

### Preferred Embodiments

### Example 1.

*In vivo* induction of TNF-α the mediator of endotoxin reaction after intravenous (i.v.) administration of the modified S-LPS and the modified lipids A of endotoxic bacteria to mice According to data from patent RU 2154068, tri-acylated and tetra-acylated lipids A of *E.coli, H. influenzae* and *P.aeruginosa* are powerful inducers of mediator of endotoxin reaction-TNF-α. Table 1 represents data extrapolated from graphs on Fig 1 (A, B, C) relating to in vivo TNF-α production in serum after i.v. administration of tri-acylated lipid A (3-acLA) of *E.coli* OM-174 and Westphal LPS *E.coli* O:111B4 to mice. Only 10-fold difference was detected for induction of TNF-α *in vivo* between *E.coli* OM-174 tri-acylated lipid A and commercially available endotoxin Westphal LPS *E.coli* O:111 B4, it is evidence that there is essential endotoxicity of E. coli tri-acetylated lipid A, excluding its use both as vaccine or vaccine component (adjuvant).

**Table 1**

| *In vivo* production of TNF-α the mediator of endotoxin reaction in blood serum after i.v. administration of E coli OM-174 tri-acylated lipid A and Westphal LPS *E.coli* O:111B4 to mice by data from patent RU 2154068 | | |
|---|---|---|
| Preparation | Dose administration to mice (mg/kg) | TNF-α concentration (pg/mL) |
| 3-acLA *E.coli* - OM174 | 0.2 | 328 |
| | 2.0 | 1644 |
| | 2.01 | 1416 |
| | 3.4 | 2000 |
| | 28.1 | 2750 |
| Westphal LPS *E.coli* 0:111B4 | 0.002 | 400 |
| | 0.020 | 1333 |
| | 0.2 | 2800 |
| | 2.0 | 4733 |
| Saline solution (0.9%-NaCl solution) | 0 | 0 |

The comparative study of *in vivo* induction of TNFa the mediator of endotoxin reaction after i.v. administration of di-acylated S-LPS (2-acLPS), tri-acylated S-LPS (3-acLPS), tetr-aacylated S-LPS (4-acLPS) and corresponding di-acylated lipids A (2-acLA), tri-acylated lipids A (3-acLA), tetra-acylated lipids A (4-acLA), obtained from actual enterobacteria *S.flexneri* 2a, *S.enterica sv typhi* O:901, *E.coli* O:55, *K.pneumoniae,* has revealed significant differences in cytokine concentration in animal sera. Obtained data are provided in Table 2, in this case the modified S-LPS and lipids of mentioned bacteria were obtained as per Example 2A. The amount of TNF-α was determined in mouse sera using test system Quantikine Mouse TNF-α/TNFSF1A (R&D Systems, USA) by ELISA according to manufacturer's standard protocol. Animal sera blood samples were taken 90 minutes after administration.

The Data presented in Table 2 prove that in contrast to the modified lipids A, corresponding modified S-LPS of enterobacteria are poor inducers of TNF-α the mediator of endotoxic shock and can be used as vaccine preparations.

**Table 2**

| TNF-α concentration (pg/mL) in serum after 2 hours after i.v. administration of the modified S-LPS and modified lipids A from enterobacteria to mice | | | | | | | | |
|---|---|---|---|---|---|---|---|---|
| Preparation/ dose administration | *S.flexneri* 2a | | S.enteric a 0:901 | *sv* typhi | *E.coli* 0:55 | | *K.pneumoniae* | |
| | S-LPS | Lipid A | S-LPS | Lipid A | S-LPS | LipidA | S-LPS | LipidA |
| | 2-acLPS 2-acLA | | 2-acLPS | 2-acLA 2-acLPS | | 2-acLA | 2-acLPS | 2-acLA |
| 50 mg/kg | 15 | 126 | 24 | 159 | 19 | 142 | 22 | 134 |
| 5 mg/kg | 13 | 75 | 19 | 101 | 18 | 57 | 11 | 53 |
| 2.5 mg/kg | 14 | 29 | 11 | 38 | 15 | 47 | 13 | 37 |
| | 3-acLPS 3-acLA | | 3-acLPS | 3-acLA 3-acLPS | | 3-acLA | 3-acLPS | 3-acLA |
| 50 mg/kg | 57 | 315 | 82 | 427 | 53 | 404 | 42 | 233 |
| 5 mg/kg | 26 | 103 | 39 | 162 | 22 | 171 | 22 | 113 |
| 2.5 mg/kg | 14 | 54 | 21 | 103 | 14 | 58 | 22 | 47 |
| | 4-acLPS 4-acLA | | 4-acLPS | 4-acLA 4-acLPS | | 4-acLA | 4-acLPS | 4-acLA |
| 50 mg/kg | 170 | 1218 | 202 | 1113 | 132 | 982 | 101 | 457 |
| 5 mg/kg | 124 | 315 | 150 | 402 | 84 | 323 | 62 | 117 |
| 2.5 mg/kg | 42 | 103 | 63 | 134 | 32 | 51 | 24 | 82 |

### Example 2

Preparation and characteristics of individual modified S-LPS of endotoxic bacteria and combinations thereof.

### A. Preparation of individual modified S-LPS of endotoxic bacteria and combinations thereof

Bacterial culture of *S.flexneri* 2a was prepared in liquid medium by deep cultivation. Separation of bacterial cells from liquid phase was performed by flow centrifuge. Obtained wet cells were washed first with saline solution then with water and then they were lyophilized.

20 g of dried bacterial cell were extracted by the Westphal method (Westphal O., Luderitz O. Chemische Erforschung von Lipopolysacchariden Gram-negativer Bakterien. Angew. Chemie., 1954, vol. 66, pp.407-17) with hot 45% -aqueous phenol at 68-70°C; 960 mg of crude LPS was obtained from aqueous phase followed by successive dialysis and lyophilisation and it then was re-dissolved in 0.05 M TRIS-buffer solution, pH = 7.2, containing 0.01 % (w/w) CaCl2 and MgCl2, RNAse and DNAse was added in concentration 100 mcg/mL and 10 mcg/mL, respectively, and after 16 hours of stirring at 37°C the reaction mixture was treated with proteinase K (20 mcg/mL) for 2 hours at 55°C. The resulting solution was dialyzed using the Vladisart installation for ultrafiltration with the limit of the passage of the membrane 50 kDa.

The dialyzed solution was concentrated and then lyophilized to give 530 mg of isolated LPS, containing not more than 2 % (w/w) of protein, determined by the Bradford method (Bradford M.M. A rapid and sensitive method for the quantitation of microgram quantities of protein utilizing the principle of protein-dye binding. Anal. Biochem., 1976, vol. 72, pp. 248-54), and not more than 2 % (w/w) of nucleic acid, determined by the Spirin method (Spirin A.S. Spectrophotometric determination of the total amount of nucleic acids. Biochemistry, 1958, v. 23, No. 4, p. 656).

Prepared LPS was characterized by SDS-PAGE data, which demonstrated the «classic» picture of a set of correctly alternating bands, each of them corresponded to S-LPS molecule with definite number of repeating units in O-specific polysaccharide chain, moreover the fastest migrating (the lowermost) zone corresponded to R-LPS (Fig.31A).

LPS isolated from dried bacterial cells *S.flexneri* 2a was dissolved in aqueous ammonia, the obtained solution was stirred on magnetic plate and then cooled to 5 - 10° C. Reaction mixture was neutralized by 10% hydrochloric acid; the obtained solution was poured into 8-10-fold volume of ethanol with stirring, precipitation was separated by centrifugation and then the precipitation procedure was repeated twice, the obtained precipitate was dissolved in alcohol and then the obtained solution was lyophilizied. Hence pure partially deacetylated S-LPS was obtained, that was proved by electrophoresis data (Fig.3; 1A). Partially deacylated S-LPS composed of di- or tri-, or tetra fatty acid residues (di-, tri- and tetra-acylated derivatives - 2-acLPS, 3-acLPS and 4-acLPS) in lipid component was prepared by varying the condition of alkaline degradation of *S.flexneri* 2a LPS, in particular, the temperature and time of saponification reaction and also concentration of ammonia hydroxide.

### B. Structure, composition and physico-chemical properties of individual modified S-LPS

Data about fatty acid composition were obtained based on the analysis of lipid A of each S-LPS extracted from each S-LPS by mild hydrolysis (1% AcOH, 100 degree, 1 hour).

Fig. 4, Fig. 5 and Fig. 6 represent typical mass-spectra of lipids A, prepared from deacylated S-LPS *S.flexneri* 2a. The characteristic signals in mass-spectra are m/z 871, 1053 and 1279 (excluding contributions of m/z). These major signals in each spectrum correspond to di-(Fig.4), tri- (Fig.5) and tetra-acylated (Fig. 6) derivatives of lipid A, respectively. It is necessary to notice a total absence of signals of penta- and hexa-acylated (m/z 1506 and 1716 respectively) derivatives in mass-spectra of deacylated lipids A, which intensity was very high in the spectra of lipid A from initial S-LPS.

For the evaluation of ratio between major and minor/minor components in the obtained products of deacylated S-LPS, the intensity of corresponding signals in mass-spectra was measured. The peaks intensity corresponding not only to mono- and diphosphorilated derivatives was taken into consideration. In the case of di-acylated S-LPS the content of the main substance was 91 % (w/w), in the case of tri-acelated one - 85 % (w/w), and in the case of tetra-acylated one - 88 % (w/w). High resolution mass-spectrometry with electrospray ionization and ion detection using ion-cyclotron resonance was performed on a Bruker Daltonics spectrometer, model Apex II, with magnet 7 (Tesla).

Also, analysis of LPS and obtained partially deacylated S-LPS was carried out by 13C-NMR spectra. 13C-NMR-spectroscopy was performed by Bruker spectrometer, model DRX-500, with XWINNMR software and impulse sequences from the manufacturer. Registration of spectra was conducted in D2O (99 : 95%) with acetone as a standard (31.5 ppm). Comparison of 13C-NMR-spectra of initial and partially deacylated S-LPS showed its full identity, except broad signal, corresponding to methylene residues of fatty acid from lipid A at the region 30-34 ppm, the integral intensity of which significantly reduced in spectra of S-LPS obtained after alkaline degradation. Additionally comparative analysis of 13C NMR-spectra demonstrates that there are no changes of primary structure of O-PS repeating units during modification (deacylation) of LPS.

Fig. 2 shows the representative 13C-NMR-spectrum of di-acylated S-LPS S. flexneri 2a, in which spectrum of region belonging to polysaccharide component is fully identical to spectrum of O-PS isolated from corresponding unmodified S-LPS. Complete coincidence of obtained spectra data with literature data (Andrei V. Perepelov, Vyacheslav L. L'vov, Bin Liu, Sof'ya N. Senchenkova, Maria E. Shekht, Alexander S. Shashkov, Lu Feng, Petr G. Aparin, Lei Wang, Yuriy A. Knirel. A similarity in the O-acetylation pattern of the Oantigens of Shigella flexneri types 1a, 1b, 2a. Carbohydr. Res., 2009, vol. 344, pp.687-92) indicates that prepared product is deacylated S-LPS with long O-PS chains, because there are no signals belong to oligosaccharide core in the spectrum.

The content of proteins and nucleic acids in obtained preparations did not exceed 1 % (w/w). Native S-LPS is extracted from dried bacterial cells *S.enterica sv typhi* or *E.coli* O:55 by abovementioned method (Example 2A) and then it was subjected to alkaline treatment to obtain pre-deacylated S-LPS, from which its individual di-, tri-, tetra-acylated were further isolated. 13C-NMR-spectrum of pre-deacylated S-LPS *S.enterica sv typhi* is reperesented on Fig. 7. Individual di-, tri- and tetra-acylated S-LPS from *K.pneumoniae* bacterium were extracted from commercially available sample of S-PLS of *K.pneumoniae* bacteria (Sigma L4288) by the same approach. Electrophoresis data (Fig.3, 2A - 4B) shows that products are S-LPS, isolated from *S.enterica sv typhi* O:901, *K.pneumoniae, E.coli* O:55.

Lyophilized substances were obtained before for the study of immunobiological properties of combinations of deacylated S-LPS of endotoxic bacteria. Substance of two-component combination was prepared by dissolution of 2-acLPS and 3-acLPS; 2-acLPS and 4-acLPS; 3-acLPS and 4-acLPS at required mass ratio in apyrogenic water, and then obtained solution was lyophilized. The substance of three-component combination was prepared containing 2-acLPS, 3-acLPS and 4-acLPS at mass ratio 1:1:1 in similar fashion.

### C. Pyrogenicity of individual modified S-LPS and combinations thereof.

Low and clinically applicable endotoxicity level of preparation of individual deacylated SLPS of endotoxic bacteria and combinations thereof was proved by special pyrogenicity studies in the experimental animals *in vivo* and by clinical studies of pyrogenicity and safety. Pyrogenicity of preparations of individual modified S-LPS - 2-acLPS, 3-acLPS, 4-acLPS and combinations thereof - (2-acLPS + 3-acLPS + 4-acLPS), (3-acLPS + 4-acLPS), (2-acLPS + 3-acLPS), (2-acLPS + 4-acLPS) of S flexneri 2a, *S.enterica sv typhi* O:901, *K.pneumoniae, E.coli* O:55 bacteria, and also LPS, obtained from *S.flexneri* 2a by the classic Westphal method was determined in comparison with commercial Vi-antigen vaccine. The test was conducted on Chinchilla rabbits weighing 2.8 - 3.05 kg in accordance with European Pharmacopoeia requirements (European Pharmacopoeia.7-th Edition, July 2010, pp. 162-163) and requirements of WHO Technical Regulations for Vi-polysaccharide vaccines (Requirements for Vi-polysaccharide typhoid vaccine. WHO Technical Report Series No.840, 1994, Annex 1). After i.v. administration of each preparation, rabbit rectal temperature was measured three times at intervals of 1 hour. A drug was considered to be apyrogenic if total temperature rise did not exceed 1.15 °C. The test results are given in Table 3. 2-acLPS S flexneri 2a and *S enterica sv* typhi O:901, *K.pneumoniae,* E. coli O:55 are highly apyrogenic preparations. In the rabbit pyrogenicity test the apyrogenic doses of administration were 34, 21, 37 and 14 mcg/kg respectively. For the di-acylated S-LPS derivative the pyrogenicity parameter greatly exceeds the requirements of WHO Committee of Experts for the polysaccharide Hib-vaccine (Recommendations for the production and control Haemophilis influenzae type b conjugate vaccines. WHO Technical Report Series, No.897, 2000). 3-acLPS is also apyrogenic product. Apyrogenic doses of administration of 3-acLPS *S.flexneri* 2a and *S.enterica sv typhi* O:901, *K pneumoniae, E.coli* O:55 were 0.2; 0.1; 0.1 and 0.1 mcg/kg respectively. Thus 3-acLPS toxicity reduction exceeds the corresponding parameters for 4-acLPS, however inferior to 2-acLPS according to the degree of detoxification.

**Table 3**

| The comparative assessment of pyrogenicity of the individual modified S-LPS and combinations thereof of *S.flexneri* 2a, *S.enterica sv typhi* O:901, *K.pneumoniae, E.coli* O:55 bacteria | | |
|---|---|---|
| Preparation | Dose administration (mcg/kg) | Pyrogenicity |
| Vi-antigen typhoid vaccine «Vianvac», lot 270 | 0.050 | apyrogenic |
| 2-acLPS *S flexneri* 2a | 34 | apyrogenic |
| 2-acLPS *S.enterica sv typhi* 0:901 | 21 | apyrogenic |
| 2-acLPS *K.pneumoniae* | 37 | apyrogenic |
| 2-acLPS *E.coli* 0:55 | 14 | apyrogenic |
| 3-acLPS *S.flexneri* 2a | 0.15 | apyrogenic |
| 3-acLPS *S.enterica sv* 0:901 | 0.1 | apyrogenic |
| 3-acLPS *K.pneumoniae* | 0.08 | apyrogenic |
| 3-acLFS *E.coli* 0:55 | 0.1 | apyrogenic |
| 4-acLPS *S.flexneri* 2a | 0.025 | apyrogenic |
| 4-acLPS *S enterica sv* Op/w 0:901 | 0.025 | apyrogenic |
| (2-acLPS + 3-acLPS + 4-acLPS) *S.flexneri* 2a at mass ratio 1:1:1 | 0.6 | apyrogenic |
| (2-acLPS +3-acLPS +4-acLPS) *S.enterica sv typhi* O:901 at mass ratio 1:1:1 | 0.4 | apyrogenic |
| (2-acLPS +3-acLPS +4-acLPS) *K.pneumoniae* at mass ratio 1:1:1 | 0.5 | apyrogenic |
| (2-acLPS +3-acLPS +4-acLPS) *E.coli* O:55 at mass ratio 1:1:1 | 0.4 | apyrogenic |
| (3-acLPS+4-acLPS) *S.flexneri* 2a at mass ratio 3 : 1 | 0.05 | apyrogenic |
| (3-acLPS + 4-acLPS) *S.enterica sv typhi* O:901 at mass ratio 3:1 | 0.05 | apyrogenic |
| (2-acLPS + 3-acLPS) *S.flexneri* 2a at mass ratio 1:1 | 0.5 | apyrogenic |
| (2-acLPS + 3-acLPS) *S.enterica sv typhi* O:901 at mass ratio 1:1 | 0.5 | apyrogenic |
| (2-acLPS + 4-acLPS) *S.flexneri* 2a at mass ratio 3:1 | 0.55 | apyrogenic |
| (2-acLPS + 4-acLPS) *S.enterica sv typhi* O:901 at mass ratio 3:1 | 0.05 | apyrogenic |
| Westphal LPS *S.flexneri* 2a | 0.0001 | apyrogenic |

The parameter of pyrogenicity of 3-acLPS meets the requirements of WHO Committee of Experts for the polysaccharide Hib-vaccine. 4-acLPS *S.flexneri* 2a and *S.enterica sv typhi* O:901 are moderately apyrogenic preparations - doses of thereof up to 0.025 mcg/kg are apyrogenic when administered intravenously. Three-component combinations of individual deacylated S-LPS - (2-acLPS + 3-acLPS + 4-acLPS) of *S. flexneri* 2a and *S.enterica sv typhi* O:901, *K. pneumoniae, E. coli* O:55 bacteria at component mass ratio 1:1:1 have exhibited low pyrogenicity in the rabbit pyrogenicity test. Doses of mentioned combinations of up to 0.6 mcg/kg of rabbit weight are apyrogenic when administered intravenously. Comparative assessment of the most sensitive parameter of *in vivo* endotoxicity reduction - pyrogenicity - demonstrates the advantage of three component combination compared with 3-acLPS and 4-acLPS which are components of this combination. The synergetic effect of their antipyrogenic action is observed at the given mass ratio: mixing of low pyrogenic 2-acLPS with more pyrogenic 3-acLPS and 4-acLPS attenuates about 3-5 times the residual endotoxicity and results in increasing of apyrogenic dose in the combinations, in particular up to 8 times in case of the 4-acLPS. It should be noted that this three-component combination have provided reasonably apparent synergistic effect at any mass ratio of the components containing in the composition, in particular, at the following mass ratio: (45:45:10); (45:10:45); (10:45:45). Two-component combinations of individual deacylated S-LPS - (2-acLPS + 3-acLPS), (2-acLPS + 4-acLPS) and (3-acLPS + 4-acLPS) of *S.flexneri* 2a and *S.enterica sv typhi* O:901, *K.pneumoniae, E.coli* O:55 bacteria also have exhibited the synergistic interaction of the components with regard to residual endotoxicity reduction of 3-acLPS or 4-acLPS at any mass ratio of the components containing in the compositions. However two-component combinations of (2-acLPS + 3-acLPS) and (2-acLPS + 4-acLPS) containing 2-acLPS not less than ½ of total weight (Table 3) have maximum synergistic effect, that allows to admit them the most promising one in terms of safety. So the combination of (2-acLPS+3-acLPS) *S.flexneri* 2a and *S.enterica sv typhi* O:901 at component mass ratio 1:1 has exhibited low pyrogenicity (dose administration to rabbits is 1.0 and 0.5 mcg/kg respectively), and combination of (2-acLPS+4-acLPS) at component mass ratio 3:1 has comparable pyrogenicity level. Specific apyrogenic dose of 3-acLPS in two component combination increases in 3 - 5 times, and of 4-acLPS - up to twice.

### D. The immunogenicity of the individual modified S-LPS and combinations thereof

Two groups of (CBAXC57B1/6) F1 mice were immunized intraperitoneally (i.p.) with preparations of the individual modified S-LPS - 2-acLPS, 3-acLPS, 4-acLPS and combinations- (2-acLPS + 3-acLPS + 4-acLPS) in component mass ratio 1:1:1, (3-acLPS + 4-acLPS) in component mass ratio 3:2, (2-acLPS + 3-acLPS) in component mass ratio 1:1, (2-acLPS + 4-acLPS) in component mass ratio 3:1 of *S.flexneri* 2a, *S.enterica sv typhi* O:901, *K.pneumoniae, E.coli* O:55 bacteria, and also with LPS, obtained from abovementioned bacteria by the classic Westphal method at a dose of 25 mcg per mouse.

At day 15 after immunization the animal blood sera samplings were taken to evaluate the S-LPS specific IgG antibody levels by ELISA method. LPS with relevant O-serotypes were used for the adsorption on microplates. To study secondary immune response the same groups of mice were immunized again at a dose of 25 mcg per mouse a month after primary injection. At day 15 after secondary immunization blood sera samplings were taken again. Obtained results show that di-acylated derivative of S-LPS from *S.flexneri* 2a and S.enterica sv typhi O:901, *K.pneumoniae, E.coli* O:55 bacteria is less immunogenic than tri- and tetra-acylated derivatives and induces low primary immune response in laboratory animals (Fig. 8A, 9A, 11A). In addition di-acylated derivative of *K.pneumoniae* induces the highest primary immune response (Fig. 10A). Level of the secondary immune IgG response in laboratory animals after immunization with 2-acLPS *S.flexneri* 2a, *S.enterica sv typhi* O:901 and E coli O:55 was significantly higher compared with the primary immune response, however, it was lower in 8.0; 8.0 and 7,4 times compared with the secondary immune response to the 3-acLPS and was lower in 12.0; 11.7 and 10.2 times compared with the secondary immune response to 4-acLPS, isolated from homological bacteria (Fig. 8B, 9B, 11B). At the same time the secondary immune IgG -response to the 2-acLPS *K.pneumoniae* was differed only in 2.4 times compared with those to the 3-acLPS (Fig. 10B).

The levels of IgG antibody production in the primary response to the 3-acLPS S. flexneri 2a, S. enterica sv typhi O:901 and E. coli O:55 were lower in 3.0; 1.1 and 3.4 times, respectively, compared with primary response to the 4-acLPS with relevant serotype (Fig. 8A, 9A, 11A). The secondary immune response after immunization of laboratory animals with 3-acLPS *S.flexneri* 2a, *S.enterica sv typhi* O:901 and *E.coli* O:55 was high (antibody titer exceeded 1600, 4000, 1800), but at the same time it was slightly lower compared with the response to 4-acLPS with relevant serotype (Fig. 8B, 9B, 11B). 4-acLPS *S.flexneri* 2a, *S enterica sv* typhi O:901 and *E.coli* O:55 induced the primary immune response in laboratory animals in 3.0; 1.1 and 3.4 times higher compared with those to the 3-acLPS (Fig. 8A, 9A, 11A). Secondary immune response level after immunization of laboratory animals with 4-acLPS was 1.4; 1.5 and 1.2 times higher compared with those to 3-acLPS with relevant serotype (Fig. 8B, 9B, 1B). A slightly higher immunogenicity of 4-acLPS antigen apparently caused by high degree of acylation of its lipid A and thereby has the pronounced ability to form aggregates compared with the other modified S-LPS, especially compared with 2-acLPS.

Combinations of individual modified S-LPS of endotoxic bacteria demonstrate high immunogenic potential despite of essential modification of the canonical structure of its lipid A domain. So three-component combination (2-acLPS + 3-acLPS + 4-acLPS) induced the primary immune response to O-antigen of *S.flexneri* 2a and S.enterica sv typhi, *K.pneumoniae, E.coli* O:55 at component mass ratio 1:1:1, level of which was rather high and did not differ significantly from the response level to classic Westphal LPS, obtained from relevant bacterial strain (Fig. 8A, 9A, 11A). Therefore the immunogenic potentials of low-endotoxic combined modified S-LPS-immunogen and endotoxic and pyrogenic LPS, prepared by the Westphal method were determined to be approximately equal, that indicates that the combination consists of special high-immunogenic with different degree of acylation associates of modified S-LPS which provided the synergistic effect to enhance of its immunogenic properties.

So three-component combinations of (2-acLPS + 3-acLPS + 4-acLPS) from *S.flexneri* 2a, *S.enterica sv typhi* O:901, *K.pneumoniae* and *E.coli* O:55 bacteria induced the secondary IgG immune response at component mass ratio 1:1:1 after immunization of laboratory animals, which was 32.0; 33.6; 8.0 and 32.0 times higher compared with the response to the 2-acLPS; was 4.0; 4.2; 3.3 and 4.3 times higher compared with the response to the 3-acLPS and was 2.7; 2.8; and 3.1 times higher compared with the response to the 4-acLPS (Fig. 8B, 9B, 11B). At the same time administered dose of the individual modified S-LPS in the composition of these combinations is only 33 % (w/w) of dose administered as an appropriate monocomponent. It should be noted that this three-component combination of S-LPS provided more or less pronounced synergistic effect directed to increasing of immunogenicity at any component mass ratio, particularly, at the following mass ratio: (10:45:45); (45:10:45); (45:45:10).

Two-component combinations of the individual modified S-LPS - (2-acLPS + 3-acLPS), (2-acLPS + 4-acLPS) and (3-acLPS + 4-acLPS) of *S.flexneri* 2a and *S.enterica sv typhi* O:901, *K.pneumoniae, E.coli* O:55 bacteria also exhibit the synergistic interaction with regard to immunogenicity enhancement at any mass ratio of the containing components. Additionally the highest secondary immune response to Salmonella or Shigella O-antigens was induced by the combinations of (2-acLPS + 3-acLPS) at component mass ratio 1:1, (3-acLPS + 4-acLPS) at component mass ratio 3:1 and (2-acLPS+4-acLPS) in component mass ratio 3:1. So IgG-response level was the highest to the combination (3-acLPS + 4-acLPS) at component mass ratio 3:1 of S. flexneri 2a and for the combination (2-acLPS + 3-acLPS) at component mass ratio 1:1 of S. enterica sv typhi O:901 (Fig. 8B, 9B). It should be noted that three-component combination provides more pronounced synergistic effect compared with to two-component combination. So slightly higher IgG-response levels were determined after immunization with three-component combination in the comparative immunogenicity study of the three-component combination from *S.flexneri* 2a and *S.enterica sv typhi* O:901 bacteria in component mass ratio 1:1:1 and two-component low-pyrogenic homologous of O-antigen combinations of (2-acLPS + 3-acLPS), (3-acLPS + 4-acLPS) and (2-acLPS+4-acLPS) at component mass ratio 1:1, 3:1 3:1, respectively (Fig. 8A, 9A).

Thus the immunogenicity of combinations of the modified S-LPS is determined by composition, amount and mass ratio of components. Three-component combination can be considered the most effective combination. In the meantime the immunogenicity of combinations of the modified S-LPS is also defined by the structure (serotype) of O-antigen of enterobacteria. For the number of serotypes the high rise of the IgG antibodies can be achieved also using two-component low-pyrogenic combinations of the modified S-LPS.

### Example 3

Vaccines containing modified S-LPS of endotoxic bacteria and combinations thereof

### A. Use of the modified S-LPS and combinations thereof in the manufacture of the unconjugated vaccine (medicament)

Preparation of unconjugated vaccine includes the synthesis of the individual di-, tri- and tetra-acylated derivatives of S-LPS and combinations as per Examples 2A, 2B and the subsequent aseptic filling of vials or syringes with solution containing the active substance and pharmaceutically acceptable special additives, which may be pH stabilizers, preservatives, adjuvants, isotonizing agents or combinations thereof. Vaccination dose contains: unconjugated form of the modified S-LPS or combination of unconjugated forms of the modified S-LPS in amount from 0.010 mg to 10 mg; phenol (preservative), not more than 0.75 mg, with addition of sodium chloride - 4.150 mg, dibasic sodium phosphate - 0.052 mg and monobasic sodium phosphate - 0.017 mg; sterile pyrogen-free water for injection - 0.5 mL (PA 42-2620-97, EP IV 2002).

### B. Pyrogenicity of the unconjugated vaccine

Pyrogenicity of vaccine containing 2-acLPS, 3-acLPS, combination of (2-acLPS +3-acLPS+4-acLPS) at component mass ratio 1:1:1, *S.flexneri* 2a; 2-acLPS, 3-acLPS, combination of (2-acLPS+3-acLPS+4-acLPS) at component mass ratio 1:1:1, S.enterica sv typhi O:901 was determined and compared with pyrogenicity of the commercial Vi-antigen vaccine. All given vaccine preparations were apyrogenic at a dose of 0.050 mcg/kg per rabbit weight. Test results are provided in Table 4.

**Table 4**

| Pyrogenicity of the unconjugated vaccine containing the modified S-LPS and combinations of the modified S-LPS of *S.flexneri* 2a and *S.enterica sv typhi* O:901 bacteria at a dose 0.050 mcg/kg per rabbit weight | | |
|---|---|---|
| Preparation | Temperature increase, in°C | Pyrogenicity |
| Vi-antigen typhoid vaccine «Vianvac» 152 | (0.3; 0.2; 0.0) ∑: 0.5 | apyrogenic |
| Dysentery vaccine containing 2-acLPS *S.flexneri* 2a | (0.2; 0.1; 0.0) ∑: 0.3 | apyrogenic |
| Dysentery vaccine containing 3-acLPS *S.flexneri* 2a | (0.2; 0.2;0.1 ∑: 0.5 | apyrogenic |
| Dysentery vaccine containing combination of (2-acLPS+3-acLPS+4-acLPS) *S.flexneri* 2a at mass ratio 1:1:1 | (0.2; 0.2; 0.1) ∑: 0.5 | apyrogenic |
| Typhoid vaccine containing 2-acLPS *S.enterica sv typhi* O:901 | (0.1; 0.2; 0.1) ∑: 0.4 | apyrogenic |
| Typhoid vaccine containing 3-acLPS *S.enterica sv typhi* O:901 | (0.2; 0.2; 0.2) ∑: 0.6 | apyrogenic |
| Typhoid vaccine containing of combination of (2-acLPS+3-acLPS + 4-acLPS) *S.enterica sv typhi* O:901 at mass ratio 1:1:1 | (0.1; 0.0; 0.3) ∑: 0.4 | apyrogenic |

### C. Protective properties of the unconjugated vaccine

The evaluation of protective properties of the vaccines containing combination of the modified S-LPS was conducted in experimental models of dysentery infection (Sereny test; Sereny B. A new method for the measurement of protective potency of disentery vaccines. Acta Microbiol., Acad.Sci. Hung., 1962, v.9, pp. 55-60) and typhoid infection (active mouseprotection test).

To study the formation of protective shigella mucosal immunity in guinea pigs, laboratory animals weighing 200-250 g were immunized twice with an interval of 10 days subcutaneously (s.c.) in the back region with vaccine, including 3-acLPS or combination of (2-acLPS+3-acLPS+4-acLPS) at mass ratio 1:1:1 or combination of (2-acLPS+3-acLPS) at mass ratio 3:1 of *S.flexneri* 2a at a doses of 25 and 50 mcg per animal. Control animals were given saline instead of the vaccine preparation. Ten days after the last immunization, Dysentery keratoconjunctivitis (Sereny test) was induced in the experimental and control animals by introduction into the eye conjunctiva cell suspension of virulent strain of *S.flexneri* 2a at a dose, close to the ID100 (10⁹ cells), and at a dose close to the 2ID100 (2x10⁹ cells), in 30 mcL of sterile saline. All animals in the control group, infected with a dose of 2x10⁹ cells, and 90% of animals in the control group, infected with a dose of 10⁹ cells, developed dysentery keratoconjunctivitis (Table 5).

Immunization with vaccine, including combination of (2-acLPS+3-acLPS+4-acLPS) *S.flexneri* 2a in mass ratio 1:1:1, at a dose of 25 mcg, provided eye protection rate in 75% of experimental animals infected at a dose of 10⁹ cells; eye protection rate was 60% when they were infected at a dose of 2x10⁹ cells. Immunization with vaccine at a dose of 50 mcg provided eye protection rate in 70% of experimental animals infected at a dose of 10⁹ cells; eye protection rate was 60% when they were infected at a dose of 2x10⁹ cells.

The eye protection rate from experimental dysentery infection when guinea pigs were immunized with vaccines, including 3-acLPS or combination of (2-acLPS+3-acLPS) at mass ratio 3:1 of *S.flexneri* 2a bacteria also varied from 55 to 75 %.

Therefore the pronounced mucosal antidysentery immunity was registered after s.c. immunization of animals with vaccine containing 3-acLPS or combination of (2-acLPS+3-acLPS+4-acLPS) at mass ratio 1:1:1 or combination of (2-acLPS+3-acLPS) at mass ratio 3:1 of *S.flexneri* 2a.

**Table 5**

| The protective mucosal immunity in guinea pigs as a result of the systemic immunization with vaccines containing the modified S-LPS and the combinations of the modified S-LPS of *S.flexneri* 2a bacteria | | | | | | | |
|---|---|---|---|---|---|---|---|
| Preparation | Preparation dose, mcg per animal | Infection dose (No. of cells in 30 mcL of saline solution) | No. of infected animals | No. of infected animal eyes | No. of eyes with kerato-conjunct i-vitis | No. of eyes protected from kerato-conjunctivitis | Eye protection rate, % |
| Vaccine, containing (2-acLPS + 3-acLPS + 4-acLPS) at mass ratio 1:1:1 | 25 | 10⁹ | 10 | 20 | 5 | 15 | 75 |
| | 25 | 2x10⁹ | 10 | 20 | 8 | 12 | 60 |
| | 50 | 10⁹ | 10 | 20 | 6 | 14 | 70 |
| | 50 | 2x10⁹ | 10 | 20 | 8 | 12 | 60 |
| Vaccine, containing (2-acLPS + 3-acLPS) at mass ratio 3:1 | 25 | 10⁹ | 10 | 20 | 5 | 15 | 75 |
| | 25 | 2x10⁹ | 10 | 20 | 7 | 13 | 65 |
| | 50 | 10⁹ | 10 | 20 | 6 | 14 | 70 |
| | 50 | 2x10⁹ | 10 | 20 | 9 | 11 | 55 |
| Vaccine, containing 3-acLPS | 25 | 10⁹ | 10 | 20 | 7 | 13 | 65 |
| | 25 | 2x10⁹ | 10 | 20 | 6 | 14 | 70 |
| | 50 | 10⁹ | 10 | 20 | 7 | 13 | 65 |
| | 50 | 2x10⁹ | 10 | 20 | 8 | 12 | 60 |
| Control | - | 10⁹ | 10 | 20 | 19 | 1 | 5 |
| | - | 2x10⁹ | 10 | 20 | 20 | 0 | 0 |

To study protective typhoid immunity the test group of (CBAXC57B1/6) F1 mice was intraperitoneally immunized with dose gradient of vaccine, including 3-acLPS *S.enterica sv typhi* O:901 and also with vaccine containing combination of (2-acLPS+3-acLPS+4-acLPS) *S.enterica sv typhi* O:901 at mass ratio 1:1:1. Control animals were given saline. After 12-14 days both animal groups were i.p. infected with 1000 cells (m.c.) of virulent typhoid strain of *S.enterica sv typhi* Ty2 No. 4446 at a dose of 80 LD50 in sterile saline containing 5 % (w/w) mucin type III (Sigma, USA) as per Joo's protocol (Joo I., Pusztai Z., Juhasz V.P. Mouseprotective ability of the international reference preparations of typhoid vaccine. Z. Immun. Forsch. exp. Ther., 1968, v.135, pp.365-72). Animal survival rate in both groups was registered for 3-5 days.

**Table 6**

| The Comparative characteristic of protective properties of vaccines, including the modified S-LPS or combination of the modified S-LPS of *S.enterica sv typhi* O:901 bacteria, and typhoid vaccine Typhim Vi | | | | |
|---|---|---|---|---|
| Preparation | Dose, mcg per mouse | Immunisation method | No of mice in group | Mouse survival rate after immunization and infection with S. enterica sv typhi Ty2 No. 4446 in 5% mucin |
| | | | | 1000 cells (80 LD50) |
| Vaccine, containing 3-acLPS typhi O:901 | 25 | i.p. | 10 | 10/10 |
| | 2.5 | i.p. | 10 | 10/10 |
| | 0.25 | i.p. | 10 | 10/10 |
| | 0.025 | i.p. | 10 | 8/10 |
| | 0.0025 | i.p. | 10 | 9/10 |
| | 0.00025 | i.p. | 10 | 8/10 |
| Vaccine, containing (2-acLPS + 3-acLPS + 4-acLPS) S.enterica sv typhi O:901 at mass ratio 1:1:1 | 25 | i.p. | 10 | 10/10 |
| | 2.5 | i.p. | 10 | 10/10 |
| | 0.25 | i.p. | 10 | 10/10 |
| | 0.025 | i.p. | 10 | 10/10 |
| | | | | |
| | 0.0025 | i.p. | 10 | 8/10 |
| | 0.00025 | i.p. | 10 | 8/10 |
| Typhim Vi | 25 | i.p. | 10 | 10/10 |
| | 2.5 | i.p. | 10 | 10/10 |
| | 0.25 | i.p. | 10 | 10/10 |
| | 0.025 | i.p. | 10 | 10/10 |
| | 0.0025 | i.p. | 10 | 10/10 |
| | 0.00025 | i.p. | 10 | 8/10 |
| Control infection | 1 cell | 10 cells | 100 cells | 1000 cells |
| LD50 = 12,5 | 8/10 | 6/10 | 0/10 | 0/10 |

As it follows from Table 6, test of active mice protection displayed the essential protective efficiency for the claimed vaccines. So vaccine, including the combination of (2-acLPS+3-acLPS+4-acLPS) *S.enterica sv typhi* O:901 at mass ratio 1:1:1 has protective efficiency comparable with vaccine preparation TYPHIM-Vi in test in mice and meets the requirements of quantitative standardization of protective activity for the typhoid vaccines. D. Anti-shock activity of unconjugated vaccine.

Animal protection from endotoxic shock was performed by prophylactic i.p. immunization of test groups of (CBAXC57B1/6)F1 mice with vaccine, including combination of (2-acLPS+3-acLPS+4-acLPS) S.sonnei at mass ratio 1:1:1 and vaccine containing combination of (2-acLPS+3-acLPS+4-acLPS) E. coli O:55 at mass ratio 1:1:1, at doses of 50, 100 and 200 mcg/ per mouse (that are equivalent to 2.5; 5 and 10 mg/kg, respectively) in 0.5 mL 0.9%-sodium chloride solution 72 hours prior to endotoxic shock induction. The endotoxic shock was induced by i.p. administration of standard endotoxin of *E.coli* O:55 (Sigma-Aldrich, USA) at a dose of 2 mg/per mouse (100 mg/kg), that is approximately 4 LD100. Control group was i.p. administered of 0.5 mL saline by the same scheme. Animal survival rate was evaluated for 3 days after injection of endotoxin (Table 7).

**Table 7**

| | | | | | | |
|---|---|---|---|---|---|---|
| The survival rate of (CBA×C57Bl/6)F1 mice, immunized by vaccines containing combinations of the modified S-LPS of S.sonnei and Escherichia coli O:55, in the induction of endotoxic shock by i.p. injection of 4 LD100 of LPS *E.coli* O:55 | | | | | | |

| Preparation | Dose, mcg per animal | No of mice in group | Death of mice at time intervals (hours) | | | Survival rate, % |
|---|---|---|---|---|---|---|
| | | | 0-24 | 24-48 | 48-72 | |
| Vaccine, containing (2-acLPS+3-acLPS+4-acLPS) S. at mass ratio 1:1:1 | 50 | 5 | 3 | --- | --- | 40 |
| | 100 | 5 | 1 | --- | --- | 80 |
| | 200 | 5 | --- | 1 | --- | 80 |
| Vaccine, containing (2-acLPS+3-acLPS+4-acLPS) E. coli O:55 at mass ratio 1:1:1 | 50 | 5 | 3 | 1 | --- | 20 |
| | 100 | 5 | 2 | 1 | --- | 40 |
| | 200 | 5 | 1 | 1 | --- | 60 |
| Control | 0 | 5 | 5 | --- | --- | 0 |

As it follows from Table 7 despite of reduced endotoxicity the claimed vaccines are effective prophylactic preparations at a dose of 100 and 200 mcg/mouse which provides 80% and 40-60%, respectively, survival rate of experimental animals associated with massive (4 LD100) endotoxic load and as a result, the correction of pathogenic mechanism of endotoxic shock. At the same time vaccine, including combination of the modified S-LPS S.sonnei had more pronounced anti shock activity than analogous *E.coli* O:55 vaccine.

Animal protection from septic shock was performed by prophylactic i.p. immunization of test groups of (CBAXC57B1/6)F1 mice with vaccine, including the combination of (2-acLPS+3-acLPS+4-acLPS) S.sonnei at mass ratio 1:1:1 and vaccine containing combination of (2-acLPS+3-acLPS+4-acLPS) E. coli O:55 at mass ratio 1:1:1, in a dose of 10 and 50 mcg per mouse (that are equivalent to 0.5 and 2.5 mg/kg, respectively) twice with an interval of 30 days prior to simulation of septic shock. Septic shock simulation was conducted after 18 days after secondary immunization. Control group consisted of intact post-operative animals (Table 8).

Septic shock (experimental peritonitis) simulation was conducted by cecal ligation and puncture procedure (CLP-model). Test and control mice groups were anesthetized by general anaesthesia, peritoneum was opened, cecum and appendix were eviscerated. The cecum was ligated in the area adjacent to appendix and punctured twice through by 22G needle. The contents of the cecum were extruded through the formed holes for contamination of the peritoneal cavity of gut contents, then organs were returned back to abdomen and abdominal cavity was stitched.

**Table 8**

| Septic shock correction in mice immunized by vaccines containing the combinations of the modified S-LPS of S.sonnei and E. coli O: 55, during the CLP-procedure on the day 8 after the primary immunization | | | | | |
|---|---|---|---|---|---|
| Preparation | Dose, mcg per mouse | Death of the first animal (hours) | Death of the last animal (hours) | Suppression of the peritonitis development Δt (hours) | Increase of survival rate under sepsis Δt (hours) |
| Vaccine containing (2-acLPS + 3-acLPS+4-acLPS) S.sonnei at mass ratio 1:1:1 | 10 | 66 | 168 | 30 | 36 |
| | 50 | 48 | 165 | 12 | 33 |
| Vaccine containing (2-acLPS+3-acLPS+4-acLPS) E. coli O:55 at mass ratio 1:1:1 | 10 | 54 | 156 | 18 | 24 |
| | 50 | 42 | 150 | 6 | 18 |
| Control | 0 | 36 | 132 | --- | ---- |

As it follows from Table 8, immunization of animals with the claimed vaccines at a dose of 10 mcg/mouse is considered to be more effective providing the suppression of experimental peritonitis development (for 30 and 18 hours compared with control group) and increase the survival rate under sepsis (for 36 and 24 hours compared with control group).

### E. Safety of unconjugated vaccine

Dysentery vaccine, including the combination of (2-acLPS+3-acLPS+4-acLPS) *S.flexneri* 2a in component mass ratio 1:1:1, and vaccine comprising the combination (2-acLPS+3-acLPS+4-acLPS) *S.enterica sv typhi* O:901 in component mass ratio 1:1:1, at a dose of 50 mcg of antigen containing in 0.5 mL phenol-phosphate buffer solution as a solvent and product for comparison - typhoid Vi-antigen vaccine "Vianvac", at a dose of 25 mcg, were injected once subcutaneously in the upper third of the shoulder to three groups of 20 adult volunteers. Temperature reactions to the drug injection, general side effects and local reactions of volunteers were studied for the first three days after immunization. Vaccine containing combination of the modified S-LPS of *S.flexneri* 2a has shown high safety profile for adult volunteers. Temperature reactions at the 37.1-37.5°C range were found in only 5% of volunteers, higher temperature reactions and general side effects were absent; local reaction (pain at the injection site) was detected only in one volunteer (Table 9). Temperature reactions in the 37.1-37.5°C range were found in only 10% of volunteers immunized with vaccine containing combination of the modified S-LPS *S.enterica sv typhi* O:901 or typhoid Vi-antigen vaccine "Vianvac" (Table 9).

**Table 9**

| Vaccine safety containing combination of the modified S-LPS *S.flexneri* 2a and S.enterica sv typhi O:901, under immunization of the adult volunteers | | | |
|---|---|---|---|
| Reactions on vaccine administration | Vaccine containing (2-acLPS+3-acLPS+4-acLPS) *S.flexneri* 2a at mass ratio 1:1:1, at a dose of 50 mcg per human (n=20) | Vaccine containing (2-acLPS+3-acLPS+4-acLPS) *S.enterica* sv *typhi* O:901 at mass ratio 1:1:1, at a dose of 50 mcg per human (n=20) | typhoid Vi-antigen vaccine "Vianvac"(lot 193), at a dose of 25 mcg per human (n=20) |
| Temperature reactions (37.1 - 37.5° C) | found in 5% of volunteers | found in 10% of volunteers | found in 10% of volunteers |
| Temperature reactions(37.6 - 38.5° C) | absent | Absent | Absent |
| Temperature reactions (38.5°C and | absent | Absent | Absent |
| General side effects | absent | Absent | Absent |
| Local reactions (pain) | 1 case | 2 cases | 1 case |

Production of proinflammatory cytokines was studied to evaluate safety of the claimed vaccines when it were administered parenterally (subcutaneously) to human. Vaccines including the combinations of modified S-LPS *S.flexneri* 2a and *S.enterica sv typhi* O:901, at a dose of 50 mcg of antigen, containing in 0.5 mL phenol-phosphate buffer solution as a solvent, were injected once subcutaneously to volunteers in the outer surface of the upper third of the shoulder.

After immunization volunteers were under the supervision of a doctor for 5 days. On the first day of supervision they underwent medical examinations at 2, 4, 6 and 24 hours after injection of drug preparations. To study cytokine status, the blood samples were taken from volunteer's veins before the injection of the claimed vaccines (0 hour) and at 2, 4 and 6 hours after administration. According to literature data TNF-α, IL-1β, IL-6 arrange triad of proinflammatory cytokines - basic mediators of sepsis (Casey L.C., Balk R.A., Bone R.C. Plasma cytokine and endotoxin levels correlate with survival in patients with the sepsis syndrome. Ann. Intern. Med., 1993; 119: 771-78.). I.v. administration of Westphal LPS *E.coli* at a dose of 0.06 - 0.2 ng/kg provides the rise of level of circulating TNF-α and IL-6 in 2-100 times (Taudorf S., Krabbe K.S., Berg R.M.G., Pedersen B.K., Moller K. Human models of low-grade inflammation: bolus versus continuous infusion of endotoxin. Clin. Vaccine Immunol., 2007; 14(3): 250-55), whereas in the experiment by immunization to volunteers with vaccine containing combination of the modified S-LPS of *S.flexneri* 2a and with typhoid vaccine containing combination of the modified S-LPS of S.enterica sv typhi O:901, in a dose of 50 mcg (that is equivalent to 0.8 -1 mcg/kg), proinflammatory cytokine concentrations were low and did not significantly differ from those in serum taken from volunteers before vaccine injection (Table 10). At the same time TNF- α concentration increased a little at 2 hours, IL-6 - at 4 hours after administration of the claimed vaccines and IL-1β concentration did not change practically and stayed at the basal level.

**Table 10**

| The profiles of proinflammatory cytokines in volunteer's blood serum after immunization with dysentery vaccine containing combination of the modified S-LPS *S.flexneri* 2a and typhoid vaccine containing combination of the modified S-LPS *S.enterica sv typhi* O:901 | | |
|---|---|---|
| Parameter | Mean value ± standard deviation (pg/mL) | |
| Time after vaccine injection (hours) | Dysentery vaccine, containing (2-acLPS+ 3-acLPS+4-acLPS) *S.flexneri* 2a at mass ratio 1:1:1, at a dose of (50 mcg/0.5 mL) | Typhoid vaccine, containing (2-acLPS+ 3-acLPS+4-acLPS) S.enterica sv typhi O:901 2a at mass ratio 1:1:1, at a dose of (50 mcg/0,5 mL) |

| TNF-a | | |
|---|---|---|
| 0 | 11.23 ± 2.2 | 13.86 ± 1.6 |
| 2 | 17.95 ± 4.34 | 17.35 ± 1.78 |
| 4 | 13.54 ± 4.18 | 11.20 ± 205 |
| 6 | 11.76 ± 0 | 10.08 ± 0 |

| IL-1β | | |
|---|---|---|
| 0 | 0.005 ± 0.009 | 0.05 ± 0 |
| 2 | 0 | 0.2 ± 0 |
| 4 | 1.49 ± 0.51 | 0.2 ± 0 |
| 6 | 0 | 0.05 ± 0 |
| IL-6 | | |
| 0 | 1.27 ± 0.91 | 10.23 ± 0.16 |

### F. Immunogenicity of unconjugated vaccine

Volunteer's immune response was investigated after single subcutaneously immunization in the upper third of the shoulder of test groups of 20 adult volunteers with vaccines including the modified S-LPS and combination of the modified S-LPS S. flexneri 2a, and also vaccine containing the combination of the modified S-LPS *S.enterica sv typhi* O:901, at a dose of 50 mcg of antigen, containing in 0.5 mL phenol-phosphate buffer solution as a solvent. Secondary immunization was performed in the same manner after 30 days after primary immunization. Blood sera for research were taken from volunteers before vaccination and after 30 days after primary and secondary vaccination, respectively.

Main classes of specific anti-LPS S. flexneri 2a and *S.enterica sv typhi* O:901 antibodies were determined by ELISA method using isotype-specific anti-IgA, anti-IgG and anti-IgM antibodies conjugated with HRP. Vaccine immunogenicity was evaluated by 4-fold-orgreater rise of levels of LPS-specific antibodies in immune sera - seroconversion compared with the background sera levels.

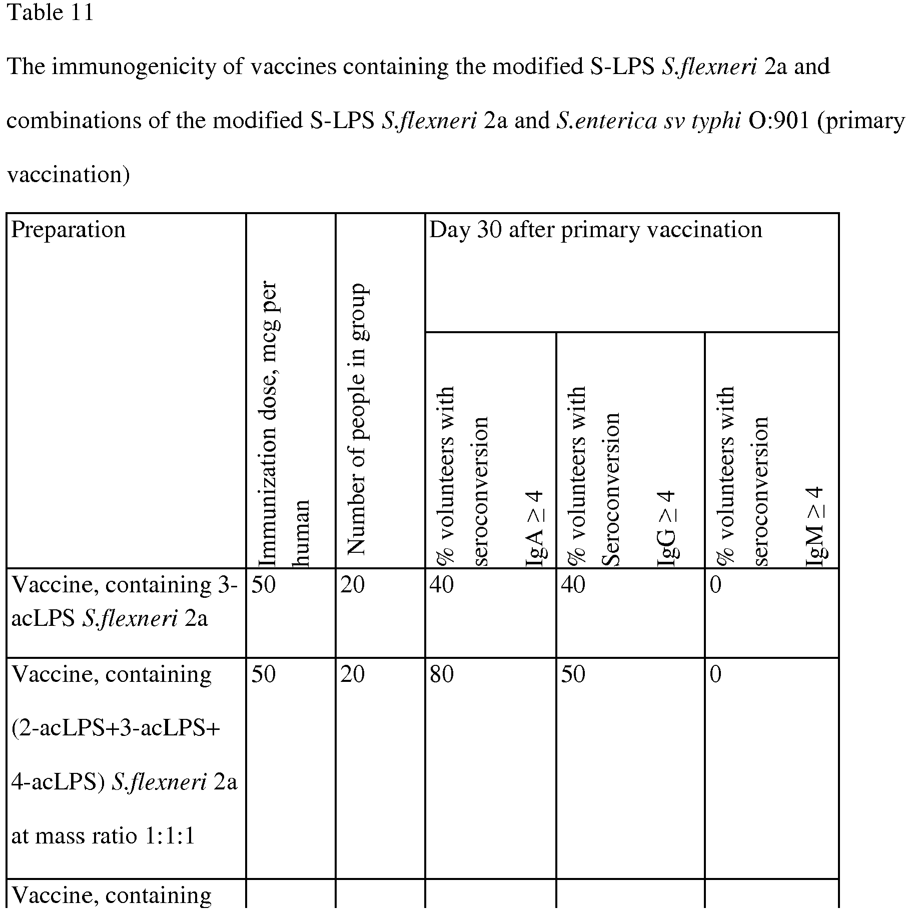

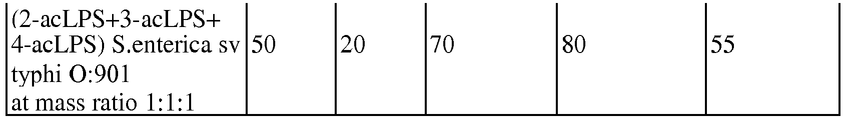

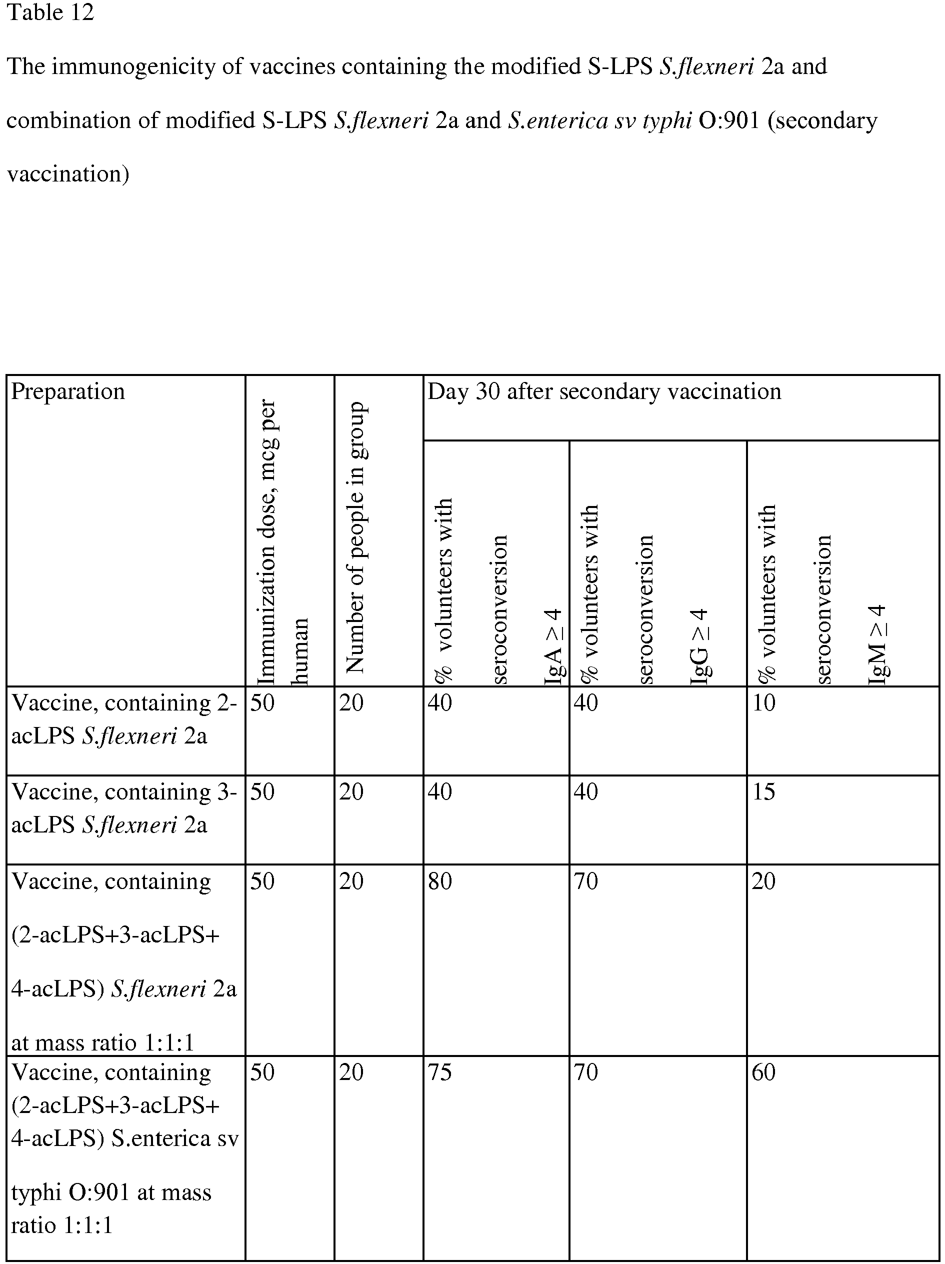

It was found that high levels of specific IgA-antibodies were observed either by detection frequency or final titer after primary and secondary immunization of volunteers with vaccines containing combinations of the modified S-LPS *S.flexneri* 2a and *S.enterica sv typhi* O:901 (Table 11, 12).

The frequency of detection of 4-fold and more rise of IgA anti-LPS antibody titers after immunization with vaccine containing combination of the modified S-LPS *S.flexneri* 2a was 80% after primary and secondary immunization, the maximum detection frequency of 4-fold Ig seroconversion of IgG antobodies in 70% cases was detected after secondary immunization.

4-fold and more titer rise of IgA anti-LPS antibodies after primary and secondary immunization with vaccine containing combination of the modified S-LPS S.enterica sv typhi O:901 was found in 70 and 75% of volunteers respectively, the maximum detection frequency of 4-fold and more seroconversions of IgG antibodies in vaccinated volunteers was 80% after primary immunization.

4-fold and more titer rise of specific IgA antibodies after primary and secondary immunization with vaccine containing 3-acLPS *S.flexneri* 2a was found in 40 and 40% of volunteers respectively. Frequency of 4-fold and more seroconversions of IgG antibodies was 40 and 40% in vaccinate volunteers after primary and secondary immunization with 3-acLPS *S.flexneri* 2a, respectively.

Level increasing of LPS-specific IgM-antibodies in volunteers immunized with vaccines including both combinations of the modified S-LPS *S.flexneri* 2a and 3-acLPS *S.flexneri* 2a was negligible and gave rise after secondary immunization to 10 and 15% of 4-fold and more seroconversions, respectively.

Frequency increasing of seroconversion of IgM-antibodies in volunteers immunized by vaccine containing combination of the modified S-LPS *S.enterica sv typhi* O:901 was significant and was 55% after primary immunization and 60% after secondary immunization. Secondary immune response was registered in volunteers immunized by vaccine including 2-acLPS *S.flexneri* 2a, as rising of IgA and IgG antibodies to LPS *S.flexneri* 2a.

Therefore trial results have proved high immunogenicity for human of dysentery vaccine, containing combination of the modified S-LPS *S.flexneri* 2a and typhoid vaccine containing combination of the modified S-LPS *S.enterica sv typhi* O:901. Specific antibodies were presented by the classes of IgA- and IgG-antibodies, the most important for mucosal immunity.

### G. Use of combinations of the modified S-LPS as immunostimulating carrier in the manufacture of conjugated vaccine (medicament)

Combination of (2-acLPS+3-acLPS+4-acLPS) at component mass ratio 1:1:1 was obtained from *S.enterica sv typhi* O:901 bacteria according to Examples 2A and 2B. To generate active functional groups in modified S-LPS, obtained combination was subjected to partial periodate oxidation followed by oxidation of generated aldehyde groups to carboxylic ones. Then partially oxidized modified S-LPS can be conjugated with vaccine antigens by any of known methods. This study used method of conjugation with polysaccharide antigen - capsule Vi-antigen or protein antigen - tetanus toxoid (TT) using 1-ethyl-3-(3-dimethylaminopropyl) carbodiimide (EDC).

Conjugation of partially oxidized combination of the modified S-LPS S.enterica sv typhi O:901 with Vi-antigen or TT was conducted in 0.2M sodium chloride solution in the presence of EDC for 4-18 hours maintaining pH value 5.6 by pH-stat. Conjugates were purified from nonconjugated initial biopolymers and low molecular impurities on Sepharose CL-6B column using 0.2M sodium chloride solution as an eluent. Fractions, containing conjugates and eluted near the column void volume, were combined for subsequent filling in sterile vials with addition of pharmaceutically suitable special additives, as which used pH stabilizers or preservatives, or adjuvants, or isotonizing agents or combinations thereof.

The vaccine conjugate of combination of the modified S-LPS *S.enterica sv typhi* O:901 with Vi-antigen contained 50% (w/w) serological active Vi-antigen determined by ELISA method. But the vaccine conjugate of the same combination with TT contained 40 % (w/w) protein mass, determined by Bradford method (Bradford M.M. A rapid and sensitive method for the quantitation of microgram quantities of protein utilizing the principle of protein-dye binding. Anal. Biochem. 1976, v. 72, pp.248-54).

One vaccination dose of conjugated vaccine contains: conjugate of combination of the modified S-LPS, from 0.010 to 0.200 mg; phenol (preservative), not more than 0.75 mg, with addition of sodium chloride - 4.150 mg, dibasic sodium phosphate, 0.052 mg and monobasic sodium phosphate, 0.017 mg; sterile pyrogen-free water for injection, 0.5 mL (PA 42-2620-97, EP IV 2002).

### H. Immunogenicity of conjugated vaccines

The mouse immune response was studied for the conjugates of polysaccharide Vi-antigen and protein TT with immunostimulating carrier - combination of (2-acLPS+3-acLPS+4-acLPS) *S.enterica sv typhi* O:901 at component mass ratio 1:1:1.

Groups of (CBAXC57Bl/6) F1 mice were i.p. immunized by abovementioned Vi-antigen conjugate and pure Vi-antigen at a dose of 25 mcg of polysaccharide per mouse. The conjugate induced humoral immune response and at 15 day after its single injection 5.8-fold rise of IgG antibody amount was detected in animal peripheral blood sera compared with the response of pure Vi-antigen by ELISA method. (Fig.12A).

To study secondary immune response the same groups of mice were re-immunized with the indicated vaccine at a dose of 25 mcg of polysaccharide per mouse one month after primary administration. On day 15 after re-immunization with conjugate 8.2-fold rise of IgG anti Vi-antibody amount was registered. (Fig. 12B).

Conjugate of protein TT with immunostimulating carrier - combination of the modified S-LPS *S.enterica sv typhi* O:901 and pure TT was also administered i.p. to groups of (CBAXC57B1/6)F1 mice at a dose of 20 mcg of protein per mouse. Conjugate induced humoral immune response after single injection and on 15 day 3-fold rise of IgG antibody amount was determined in animal peripheral blood sera compared with the response of pure TT (Fig.12A).

To study secondary immune response the same groups of mice were re-immunized with the indicated vaccine at a dose of 20 mcg of protein per mouse after month after primary administration. On day 15 after re-immunization with conjugate 6.4-fold rise of IgG anti-TT antibody amount was registered. (Fig.12B).

It should be noted that O-specific antibody levels to immunostimulating carrier - combination of the modified S-LPS *S.enterica sv typhi* O:901, induced by conjugate with TT, also rose: at primary response - in 3.2 times and at secondary one -1.5 times (Fig. 12A and B). Therefore additional rise of immune response was observed both for protein antigen and for carrier when administered of S-LPS conjugate with protein carrier.

### I. Multivalent Dysentery-Typhoid-Escherichia coli Vaccine

To prepare polyvalent vaccine substance against Shigella flexneri or sonnei shigelosis, typhoid fever and enterohaemorrhagic *E.coli* O:55 infection equal mass fractions of combination (2-acLPS+3-acLPS+4-acLPS) substance at component ratio 1:1:1 of serotype *S.flexneri* 2a, S. sonnei, *S.enterica sv typhi* O:901 and *E.coli* O:55, obtained as per Examples 2A and 2B were dissolved in pyrogen-free water. Final vaccine form was prepared according to Example 3A.

The immunogenicity of multivalent Dysentery - Typhoid-Escherichia coli vaccine was determined by tests in (CBAXC57B1/6)FI mice, which were immunized i.p. by polyvalent vaccine at a dose of 100 mcg per mouse. Components of polyvalent vaccine - combination (2-acLPS+3-acLPS+4-acLPS) at ratio 1:1:1 *S.flexneri* 2a or S. sonnei or S.enterica sv typhi O:901 or *E.coli* O:55 were also injected separately to different mouse groups at a dose of 25 mcg per mouse. On 15 day animal blood sera samplings were taken. To study secondary immune response the same groups of mice were re-immunized by the indicated drug preparations at a dose of 100 or 25 mcg per mouse one month after primary injection. On day 15 after secondary immunization animal blood sera samplings were taken. Multivalent vaccine induced immune response after primary and secondary immunization. At the same time multivalent vaccine exceeded IgG antibody titer for the all its individual components - typhoid *S.enterica sv typhi* O:901, dysentery S. flexneri 2a and dysentery S.sonnei and Escherichia coli O:55 up to level compared with response level after separate administration of the corresponding component at a dose of 25 mcg (Fig. 13A, B).

Thus multivalent Dysentery-Typhoid-Escherichia coli vaccine simultaneously induces immune response in mice to the modified S-LPS antigens, isolated from 4 bacteria strains, relating to three different families of endotoxic bacteria.

### Example 4

### Pharmaceutical composition containing the modified S-LPS and combinations of them

### A. Use of the modified S-LPS and combinations of them in the manufacture of the pharmaceutical composition (medicament)

Preparation of pharmaceutical composition includes the synthesis of the modified S-LPS and combinations thereof as per Examples 2A and 2B with the subsequent aseptic filling of vials or syringes with solution containing the active substance and pharmaceutically suitable special additives, as which may be used pH stabilizers, preservatives, adjuvants, isotonizing agents or combinations thereof. Therapeutic dose of pharmaceutical composition contains: combination of (2-acLPS+3-acLPS+4-acLPS) at component mass ratio 1:1:1, S.enterica sv typhi O:901 salmonella, from 0.010 to 50.000 mg; phenol (preservative), not more than 0.75 mg, with addition of sodium chloride - 4.150 mg and monobasic sodium phosphate, 0.017 mg; sterile pyrogen-free water for injection, 0.5 mL (PA 42-2620-97, EP IV 2002).

### B. Antiviral action of pharmaceutical composition

Antiviral action of pharmaceutical composition containing combination of (2-acLPS+3-acLPS+4-acLPS) at component mass ratio 1:1:1 from *S.enterica sv typhi* O:901 salmonella was studied in white mice. The test and control groups of male mice (per 10 animals in group) weighing 18-20 g were infected with virulent influenza A H1N1 virus at a dose of LD100, after this animals in test groups were treated by i.p. daily administration of the drug preparation composed of combination of (2-acLPS+3-acLPS+4-acLPS) at component mass ratio 1:1:1 *S.enterica sv typhi* O:901, at a dose of 100 mcg per animal. Control groups of mice were given saline solution in similar fashion. Animal survival rate was determined for two weeks after infection. Mice survival rate was 0% in control group and 40% in test group (Fig. 14). At the same time the average life expectancy of test groups were statistically-valid higher (p > 0.001) than in control ones. Therefore obtained experimental data prove that the claimed pharmaceutical composition has the effect of modulating of immune system reactions.

### C. The tolerogenic effect of pharmaceutical composition

Test groups of (CBA×C57Bl/6)FI mice were immunized i.p. with pharmaceutical compositions containing 2-acLPS *S.enterica sv typhi* O:901 or 3-acLPS S.enterica sv typhi O:901, or combination of (2-acLPS+3-acLPS+4-acLPS) at mass ratio 1:1:1 S.enterica sv typhi O:901, at a dose of 50, 100 and 200 mcg/ mouse, respectively (that are equivalent to 2.5; 5 and 10 mg/kg) in 0.5 mL of 0.9% sodium chloride (saline solution) prior 72 hours to injection of standard endotoxin-LPS *E.coli* O:55 (Sigma-Aldrich, USA) at a dose of 2 mg/mouse (that is equivalent to 100 mg/kg), that is LD100. Control group of mice was injected i.p. 0.5 mL of saline by the same scheme.

TNF-α amount was determined in mouse blood sera with test-system Quantikine Mouse TNF-α/TNFSF1A (R&D Systems, USA) by ELISA method according to manufacturer's standard protocol. Blood was taken from animals after 90 minutes after endotoxic shock induction. Test results are presented in Table 13.

**Table 13**

| The TNF-α production in mice after pre-administration of the claimed pharmaceutical composition performed 72 hours before endotoxic shock induction | | |
|---|---|---|
| Preparation | Dose, mcg/mouse | TNF-a, (pg/mL) |
| Pharmaceutical composition | 50 | 488 |
| | 100 | 475 |
| | 200 | 468 |
| Pharmaceutical composition | 50 | 483 |
| | 100 | 413 |
| | 200 | 374 |
| Pharmaceutical composition | 50 | 493 |
| | 100 | 448 |
| | 200 | 397 |
| Control | 0 | 915 |

Pre-administration of mice with the claimed pharmaceutical composition provided the reduction of *in vivo* TNF-α production by macrophage to a level below 500 pg/mL while in control group the same level was more than 900 pg/mL (Table 13). Dose-dependent suppression of TNF-α production under immunization with the claimed pharmaceutical compositions proves their tolerogenic effect, which can be used for the correction of various pathological states associated with hyperproduction of proinflammatory cytokines.

## Claims

1. A modified lipopolysaccharide (S-LPS) of endotoxic bacteria, wherein said modified lipopolysaccharide is free of lipopolysaccharides containing lipid A having five acyl residues or six acyl residues, comprising:
a) O-specific polysaccharide antigen consisting of one or more repeating units;
b) core oligosaccharide; and
c) either
(i) fully O-deacylated lipid A containing two acyl residues; or
(ii) partially O-deacylated lipid A containing three or four acyl residues.

2. The modified lipopolysaccharide of claim 1, which is a lipopolysaccharide comprising lipid A containing two acyl residues.

3. The modified lipopolysaccharide of claim 2 which is at least 85% pure.

4. The modified lipopolysaccharide (S-LPS) of endotoxic bacteria of claim 1, wherein the modified lipopolysaccharide (S-LPS) is a lipopolysaccharide comprising lipid A containing three acyl residues.

5. The modified lipopolysaccharide (S-LPS) of claim 4, which is at least 80% pure.

6. The modified lipopolysaccharide (S-LPS) of endotoxic bacteria of claim 1, wherein the modified lipopolysaccharide (S-LPS) is a lipopolysaccharide comprising lipid A containing four acyl residues.

7. The modified lipopolysaccharide of claim 6 which is at least 80% pure.

8. The modified lipopolysaccharide of any one of claims 1 to 7, which is lipopolysaccharide of endotoxic bacteria selected from the group comprising *Salmonella, Escherichia, Shigella, Bordetella, Haemophilus, Neisseria, Campylobacter, Vibrio, Klebsiella, Chlamydya, Corynobacterium* and combinations thereof.

9. A combination of the modified lipopolysaccharides, wherein said modified lipopolysaccharide is free of lipopolysaccharides containing lipid A having five acyl residues or six acyl residues, that comprises (a) a modified lipopolysaccharide (S-LPS) of endotoxic bacteria comprising: O-specific polysaccharide antigen consisting of one or more repeating units, core oligosaccharide and fully O-deacylated lipid A containing two acyl residues, and (b) a modified lipopolysaccharide (S-LPS) of endotoxic bacteria comprising: O-specific polysaccharide antigen consisting of one or more repeating units, core oligosaccharide and partially O-deacylated lipid A containing three acyl residues.

10. A combination of the modified lipopolysaccharides, wherein said modified lipopolysaccharide is free of lipopolysaccharides containing lipid A having five acyl residues or six acyl residues, that comprises (a) a modified lipopolysaccharide (S-LPS) of endotoxic bacteria comprising: O-specific polysaccharide antigen consisting of one or more repeating units, core oligosaccharide and fully O-deacylated lipid A containing two acyl residues, (b) a modified lipopolysaccharide (S-LPS) of endotoxic bacteria comprising: O-specific polysaccharide antigen consisting of one or more repeating units, core oligosaccharide and partially O-deacylated lipid A containing three acyl residues, and (c) a modified lipopolysaccharide (S-LPS) of endotoxic bacteria that comprises: O-specific polysaccharide antigen consisting of one or more repeating units, core oligosaccharide and partially O-deacylated lipid A containing four acyl residues.

11. A combination of modified lipopolysaccharides, wherein said modified lipopolysaccharide is free of lipopolysaccharides containing lipid A having five acyl residues or six acyl residues, comprising (a) a modified lipopolysaccharide (S-LPS) of endotoxic bacteria, comprising: O-specific polysaccharide antigen consisting of one or more repeating units, core oligosaccharide and fully O-deacylated lipid A containing two acyl residues, and (b) a modified lipopolysaccharide (S-LPS) of endotoxic bacteria, comprising: O-specific polysaccharide antigen consisting of one or more repeating units, core oligosaccharide and partially O-deacylated lipid A containing four acyl residues.

12. A combination of the modified lipopolysaccharides, wherein said modified lipopolysaccharide is free of lipopolysaccharides containing lipid A having five acyl residues or six acyl residues, that comprises (a) a modified lipopolysaccharide (S-LPS) of endotoxic bacteria, comprising: O-specific polysaccharide antigen consisting of one or more repeating units, core oligosaccharide and partially O-deacylated lipid A containing three acyl residues, and (b) a modified lipopolysaccharide (S-LPS) of endotoxic bacteria, comprising: O-specific polysaccharide antigen consisting of one or more repeating units, core oligosaccharide and partially O-deacylated lipid A containing four acyl residues.

13. A composition containing the modified lipopolysaccharide (S-LPS) of endotoxic bacteria of any one of claims 1 to 8 or the combination of modified lipopolysaccharides of any one of claims 9 to 12 for use in the treatment or prophylaxis of septic shock or endotoxic shock or in the treatment of influenza or for use as a therapeutic or preventative vaccine for bacterial or viral infection.

14. A vaccine comprising the modified lipopolysaccharide (S-LPS) of endotoxic bacteria of any one of claims 1 to 8 or the combination of modified lipopolysaccharides of any one of claims 9 to 12.

15. A pharmaceutical composition comprising the modified lipopolysaccharide (S-LPS) of endotoxic bacteria of any one of claims 1 to 8 or the combination of modified lipopolysaccharides of any one of claims 9 to 12.

## Patentansprüche

1. Modifiziertes Lipopolysaccharid (S-LPS) endotoxischer Bakterien, wobei das modifizierte Lipopolysaccharid frei von Lipopolysacchariden ist, die Lipid A mit fünf Acylresten oder sechs Acylresten enthalten, Folgendes umfassend:
a) O-spezifisches Polysaccharidantigen, bestehend aus einer oder mehreren sich wiederholenden Einheiten;
b) Kernoligosaccharid; und
c) entweder
(i) vollständig O-deacyliertes Lipid A, zwei Acylreste enthaltend; oder
(ii) teilweise O-deacyliertes Lipid A, drei oder vier Acylreste enthaltend.

2. Modifiziertes Lipopolysaccharid nach Anspruch 1, bei dem es sich um ein Lipopolysaccharid handelt, das Lipid A umfasst, welches zwei Acylreste enthält.

3. Modifiziertes Lipopolysaccharid nach Anspruch 2, das zu mindestens 85 % rein ist.

4. Modifiziertes Lipopolysaccharid (S-LPS) endotoxischer Bakterien nach Anspruch 1, wobei das modifizierte Lipopolysaccharid (S-LPS) ein Lipopolysaccharid ist, das Lipid A umfasst, welches drei Acylreste enthält.

5. Modifiziertes Lipopolysaccharid (S-LPS) nach Anspruch 4, das zu mindestens 80 % rein ist.

6. Modifiziertes Lipopolysaccharid (S-LPS) endotoxischer Bakterien nach Anspruch 1, wobei das modifizierte Lipopolysaccharid (S-LPS) ein Lipopolysaccharid ist, das Lipid A umfasst, welches vier Acylreste enthält.

7. Modifiziertes Lipopolysaccharid nach Anspruch 6, das zu mindestens 80 % rein ist.

8. Modifiziertes Lipopolysaccharid nach einem der Ansprüche 1 bis 7, welches ein Lipopolysaccharid endotoxischer Bakterien ist, ausgewählt aus der Gruppe bestehend aus Salmonella, Escherichia, Shigella, Bordetella, Haemophilus, Neisseria, Campylobacter, Vibrio, Klebsiella, Chlamydya, Corynobacterium sowie Kombinationen daraus.

9. Kombination aus den modifizierten Lipopolysacchariden, wobei das modifizierte Lipopolysaccharid frei von Lipopolysacchariden ist, die Lipid A mit fünf Acylresten oder sechs Acylresten enthalten, umfassend (a) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und vollständig O-deacyliertes Lipid A, das zwei Acylreste enthält, und (b) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und teilweise O-deacyliertes Lipid A, das drei Acylreste enthält.

10. Kombination aus den modifizierten Lipopolysacchariden, wobei das modifizierte Lipopolysaccharid frei von Lipopolysacchariden ist, die Lipid A mit fünf Acylresten oder sechs Acylresten enthalten, umfassend (a) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und vollständig O-deacyliertes Lipid A, das zwei Acylreste enthält, (b) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und teilweise O-deacyliertes Lipid A, das drei Acylreste enthält, und (c) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und teilweise O-deacyliertes Lipid A, das vier Acylreste enthält.

11. Kombination aus modifizierten Lipopolysacchariden, wobei das modifizierte Lipopolysaccharid frei von Lipopolysacchariden ist, die Lipid A mit fünf Acylresten oder sechs Acylresten enthalten, umfassend (a) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und vollständig O-deacyliertes Lipid A, das zwei Acylreste enthält, und (b) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und teilweise O-deacyliertes Lipid A, das vier Acylreste enthält.

12. Kombination aus den modifizierten Lipopolysacchariden, wobei das modifizierte Lipopolysaccharid frei von Lipopolysacchariden ist, die Lipid A mit fünf Acylresten oder sechs Acylresten enthalten, umfassend (a) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und teilweise O-deacyliertes Lipid A, das drei Acylreste enthält, und (b) ein modifiziertes Lipopolysaccharid (S-LPS) endotoxischer Bakterien, umfassend: O-spezifisches Polysaccharidantigen, bestehend aus einer oder mehreren sich wiederholenden Einheiten, Kern-Oligosaccharid und teilweise O-deacyliertes Lipid A, das vier Acylreste enthält.

13. Zusammensetzung, das modifizierte Lipopolysaccharid (S-LPS) endotoxischer Bakterien nach einem der Ansprüche 1 bis 8 oder die Kombination aus modifizierten Lipopolysacchariden nach einem der Ansprüche 9 bis 12 enthaltend, zur Verwendung bei der Behandlung oder zur Vorbeugung von septischem Schock oder endotoxischem Schock oder bei der Behandlung von Influenza oder zur Verwendung als Therapeutikum oder als vorbeugendes Impfmittel für bakterielle oder virale Infektion.

14. Impfmittel, das modifizierte Lipopolysaccharid (S-LPS) endotoxischer Bakterien nach einem der Ansprüche 1 bis 8 oder die Kombination aus modifizierten Lipopolysacchariden nach einem der Ansprüche 9 bis 12 enthaltend.

15. Pharmazeutische Zusammensetzung, das modifizierte Lipopolysaccharid (S-LPS) endotoxischer Bakterien nach einem der Ansprüche 1 bis 8 oder die Kombination aus modifizierten Lipopolysacchariden nach einem der Ansprüche 9 bis 12 enthaltend.

## Revendications

1. Lipopolysaccharide modifié (S-LPS) de bactéries endotoxiques, ledit lipopolysaccharide modifié étant exempt de lipopolysaccharides contenant le lipide A possédant cinq radicaux acyle ou six radicaux acyle, comprenant :
a) un antigène de polysaccharide O-spécifique constitué d'un ou plusieurs motifs répétitifs ;
b) un noyau oligosaccharide ; et
c) soit
(i) le lipide A totalement O-déacétylé contenant deux radicaux acyle ; soit
(ii) le lipide A partiellement O-déacétylé contenant trois ou quatre radicaux acyle.

2. Lipopolysaccharide modifié selon la revendication 1, qui est un lipopolysaccharide comprenant le lipide A contenant deux radicaux acyle.

3. Lipopolysaccharide modifié selon la revendication 2 qui est au moins 85 % pur.

4. Lipopolysaccharide modifié (S-LPS) de bactéries endotoxiques selon la revendication 1, le lipopolysaccharide modifié (S-LPS) étant un lipopolysaccharide comprenant le lipide A contenant trois radicaux acyle.

5. Lipopolysaccharide modifié (S-LPS) selon la revendication 4, qui est au moins 80 % pur.

6. Lipopolysaccharide modifié (S-LPS) de bactéries endotoxiques selon la revendication 1, le lipopolysaccharide modifié (S-LPS) étant un lipopolysaccharide comprenant le lipide A contenant quatre radicaux acyle.

7. Lipopolysaccharide modifié selon la revendication 6, qui est au moins 80 % pur.

8. Lipopolysaccharide modifié selon l'une quelconque des revendications 1 à 7, qui est un lipopolysaccharide de bactéries endotoxiques choisi dans le groupe comprenant *Salmonella, Escherichia, Shigella, Bordetella, Haemophilus, Neisseria, Campylobacter, Vibrio, Klebsiella, Chlamydya, Corynobacterium* et des combinaisons correspondantes.

9. Combinaison de lipopolysaccharides modifiés, ledit lipopolysaccharide modifié étant exempt de lipopolysaccharides contenant le lipide A possédant cinq radicaux acyle ou six radicaux acyle, qui comprend (a) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A totalement O-déacétylé contenant deux radicaux acyle, et (b) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A partiellement O-déacétylé contenant trois radicaux acyle.

10. Combinaison de lipopolysaccharides modifiés, ledit lipopolysaccharide modifié étant exempt de lipopolysaccharides contenant le lipide A possédant cinq radicaux acyle ou six radicaux acyle, qui comprend (a) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A totalement O-déacétylé contenant deux radicaux acyle, et (b) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A partiellement O-déacétylé contenant trois radicaux acyle, et (c) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A partiellement O-déacétylé contenant quatre radicaux acyle.

11. Combinaison de lipopolysaccharides modifiés, ledit lipopolysaccharide modifié étant exempt de lipopolysaccharides contenant le lipide A possédant cinq radicaux acyle ou six radicaux acyle, qui comprend (a) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A totalement O-déacétylé contenant deux radicaux acyle, et (b) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A partiellement O-déacétylé contenant quatre radicaux acyle.

12. Combinaison de lipopolysaccharides modifiés, ledit lipopolysaccharide modifié étant exempt de lipopolysaccharides contenant le lipide A possédant cinq radicaux acyle ou six radicaux acyle, qui comprend (a) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A partiellement O-déacétylé contenant trois radicaux acyle, et (b) un lipopolysaccharide modifié (S-LPS) de bactéries endotoxiques comprenant : un antigène de polysaccharide O-spécifique constitué d'un ou plusieurs motifs répétitifs, un noyau oligosaccharide et le lipide A partiellement O-déacétylé contenant quatre radicaux acyle.

13. Composition contenant le lipopolysaccharide modifié (S-LPS) de bactéries endotoxiques selon l'une quelconque des revendications 1 à 8 ou la combinaison de lipopolysaccharides modifiés selon l'une quelconque des revendications 9 à 12 pour une utilisation dans le traitement ou la prophylaxie d'un choc septique ou d'un choc endotoxique ou dans le traitement de la grippe ou pour une utilisation en tant que vaccin thérapeutique ou préventif contre une infection bactérienne ou virale.

14. Vaccin comprenant le lipopolysaccharide modifié (S-LPS) de bactéries endotoxiques selon l'une quelconque des revendications 1 à 8 ou la combinaison de lipopolysaccharides modifiés selon l'une quelconque des revendications 9 à 12.

15. Composition pharmaceutique comprenant le lipopolysaccharide modifié (S-LPS) de bactéries endotoxiques selon l'une quelconque des revendications 1 à 8 ou la combinaison de lipopolysaccharides modifiés selon l'une quelconque des revendications 9 à 12.
